# EUROPEAN PATENT SPECIFICATION

(11) **EP 2 718 714 B1**
(45) Date of publication and mention of the grant of the patent: **29.07.2015**
(21) Application number: 12732994.4
(22) Date of filing: 11.06.2012
(51) Int. Cl.: G01N 33/50

(54) **MARKERS FOR IMPAIRED BONE FRACTURE HEALING**
MARKER FÜR BEEINTRÄCHTIGTE KNOCHENBRUCHHEILUNG
MARQUEURS POUR LA GUÉRISON DE FRACTURES OSSEUSES ALTÉRÉES

(30) Priority: 10.06.2011 EP 11169591
(43) Date of publication of application: 16.04.2014
(73) Proprietor: Université Libre de Bruxelles, 1050 Bruxelles (BE); Bone Therapeutics S.A., 6041 Gosselies (BE); Université de Liège, 4000 Liège (BE); Centre Hospitalier Universitaire de Liège, 4000 Liège (BE)
(72) Inventor: GANGJI, Valérie, B-1640 Rhode-Saint-Genese (BE); HAUZEUR, Jean-Philippe, B-1200 Bruxelles (BE); DE SENY, Dominique, B-4000 Liège (BE); MATHIEU, Myrielle, B-1700 Dilbeek (BE); INGELS, Aude, B-1348 Louvain-La-Neuve (BE); RIGUTTO, Sabrina, B-1570 Tollembeek (BE); SPRUYT, Delphine, B-6060 Gilly (BE); BASTIANELLI, Enrico, B-1640 Rhode-Saint-Genese (BE); ALBARANI, Valentina, B-1160 Bruxelles (BE); PESESSE, Xavier, B-1430 Bierghes (BE); MALAISE, Michel, B-4030 Grivegnée (BE)
(74) Representative: Michalík, Andrej
(86) International application number: PCT/EP2012/061034
(87) International publication number: WO 2012/168482

(56) References cited:
- C.A. BEETON ET AL: "Circulating levels of interleukin-6 and its soluble receptor in patients with head injury and fracture", JOURNAL OF BONE AND JOINT SURGERY - BRITISH VOLUME, vol. 86, no. 6, 1 August 2004 (2004-08-01) , pages 912-917, XP055012234, ISSN: 0301-620X, DOI: 10.1302/0301-620X.86B6.14176
- TOSHIYUKI KITAORI ET AL: "Stromal cell-derived factor 1/CXCR4 signaling is critical for the recruitment of mesenchymal stem cells to the fracture site during skeletal repair in a mouse model", ARTHRITIS & RHEUMATISM, vol. 60, no. 3, 1 March 2009 (2009-03-01), pages 813-823, XP055004135, ISSN: 0004-3591, DOI: 10.1002/art.24330
- KIDD L J ET AL: "Temporal pattern of gene expression and histology of stress fracture healing", BONE, PERGAMON PRESS., OXFORD, GB, vol. 46, no. 2, 1 February 2010 (2010-02-01), pages 369-378, XP026864582, ISSN: 8756-3282 [retrieved on 2009-10-15]
- CAETANO-LOPES J ET AL: "Upregulation of inflammatory genes and downregulation of sclerostin gene expression are key elements in the early phase of fragility fracture healing", PLOS ONE 2011 PUBLIC LIBRARY OF SCIENCE USA LNKD- DOI:10.1371/JOURNAL.PONE.0016947, vol. 6, no. 2, 2011, XP009158233, ISSN: 1932-6203

## Description

### FIELD OF THE INVENTION

The invention relates to biomarkers and parameters useful for the diagnosis, prediction, prognosis and/or monitoring of diseases and conditions in subjects, in particular impaired bone fracture healing, such as but not limited to non-union fractures, mal-union fractures or delayed union fractures; and to related methods, uses, kits and devices.

### BACKGROUND OF THE INVENTION

There exists a continuous need for additional and preferably improved ways to obtain accurate prediction, diagnosis, prognosis and/or monitoring of diseases and conditions in subjects in order to inform and guide treatment choices.

Impaired fracture healing encompasses any anomalies and deficiencies of bone fracture healing such as inadequate, delayed or absent bone fracture healing, including without limitation mal-unions, delayed unions and non-unions. Non-union fractures, also known as non-unions (NU), including *inter alia* tight non-unions and unstable non-unions (pseudarthrosis), are characterised by a failure of fracture repair processes, without hope of spontaneous healing. The reported rate of non-unions varies between 2% and 10% of all fractures, depending on the authors (Gaston et al. J. Bone Joint Surg. Br., 2007, vol. 89(12), 1553-1560; Tzioupis and Giannoudis. Injury, 2007, vol. 38 Suppl 2, S3-S9). Non-unions may be classified as hypertrophic or oligotrophic if bony fragment sites are vascular. Hypertrophic non-unions are usually explained by an instability at the fracture site. The oligotrophic non-unions typically occur after major displacement of the fracture sites and present an inadequate healing response as shown by the absence of callus. In non-unions classified as atrophic, the bony fragments are avascular, adynamic and incapable of biologic reaction (Frolke et al. Injury, 2007, vol. 38 Suppl 2, S19-S22).

Mal-unions are characterized by an imperfect union of previously fragmented bone. A delayed union can be defined as a fracture in which healing has not occurred in the expected time and the outcome remains uncertain.

In normal healing process, a bone fracture initiates a sequence of inflammation, repair, and remodelling that can restore the injured bone to its original state. In humans, the inflammatory phase lasts about 5 to 7 days and begins with the development of a haematoma and is followed by the invasion of inflammatory cells. These cells, in association with the local cells, secrete cytokines, chemokines and growth factors to promote the recruitment of osteogenic progenitor cells and endothelial progenitor cells, essential to initiate the repair process (Einhorn. Clin. Orthop. Relat. Res., 1998, vol. 355 Suppl: S7-21). The recruitment of progenitor cells is divided in four phases: mobilisation, migration, invasion and engraftment of the cells to the fracture site. Impairment of *inter alia* any one or more of the above processes can result in impaired bone fracture healing.

The diagnosis is currently radiologic and is made after 3 to 4 months without consolidation for delayed unions and after 6 to 9 months without consolidation for non-unions, depending on the site and type of fracture (Frolke et al. *Supra*)*.* Clinically useful, biomarker-based screening tests for impaired bone fracture healing are, to our knowledge, not available. In fact, the use of biological molecules as biomarkers for impaired bone fracture healing has never been investigated in non-union patients (NU). In pathophysiological studies, authors have demonstrated that *in situ* TGF-β, PDGF, FGF, and BMP 2/4 levels were the same in NU and healthy subjects 1 week after trauma, but were decreased 8 weeks later in the NU patients (Brownlow et al. Injury, 2001, vol. 32(7), 519-524). Other authors have shown that serum levels of TGF-β, PDGF-AB and FGF were lower at 2 and 4 weeks after trauma in NU patients. TGF-β1 and PDGF-AB were still lower in NU patients at 12 weeks after trauma (Weiss et al. Arch. Orthop. Trauma Sung., 2009, vol. 129, 989-997; Zimmermann et al. Bone, 2005, vol. 36(5), 779-785).

Because knowledge, indication or warning that a fracture in a subject is or has an increased chance to show impaired healing, such as to progress towards a non-union, may aid therapeutic interventions in the subject, the provision of further, alternative and preferably improved methods and means for diagnosis, prediction, prognosis and/or monitoring of impaired bone fracture healing continues to be of prime importance.

### SUMMARY OF THE INVENTION

Having conducted extensive experiments and tests, the inventors identified biological molecules whose levels are closely predictive and/or indicative of impaired healing of a bone fracture, and which thus constitute useful and promising biomarkers for impaired fracture healing. The synonymous phrases "impaired bone fracture healing" or "impaired fracture healing" as used herein encompass any anomalies, abnormalities and deficiencies of bone fracture healing, such as inadequate, delayed or absent bone fracture healing. The phrases intend to specifically comprise and preferably denote mal-unions, delayed unions and non-unions, more preferably denote non-unions, including *inter alia* tight non-unions and unstable non-unions (pseudarthrosis).

In accordance with the invention, additional and markedly improved methods and means for diagnosis, prediction, prognosis and/or monitoring of impaired fracture healing are realised through the provision of any one or more of stromal derived factor-1 (SDF-1 or CXCL12), platelet-derived growth factor BB (PDGF-BB), interleukin-8 (IL-8 or CXCL8) and interleukin-6 (In-6) as biomarker(s) for impaired bone fracture healing.

Hence, provided herein is use of any one or more of SDF-1, PDGF-BB, IL-8 and IL-6 as a biomarker, more particularly as a biomarker for impaired fracture healing, even more particularly as a biomarker for the diagnosis, prediction, prognosis and/or monitoring of impaired fracture healing. Preferably, provided herein is use of IL-8 as a biomarker, more particularly as a biomarker for impaired bone fracture healing, even more particularly as a biomarker for the diagnosis, prediction, prognosis and/or monitoring of impaired bone fracture healing.

Also provided is use of any one or more of SDF-1, PDGF-BB, IL-8 and IL-6 for the diagnosis, prediction, prognosis and/or monitoring of impaired fracture healing. Preferably, provided herein is use of IL-8 for the diagnosis, prediction, prognosis and/or monitoring of impaired bone fracture healing.

In preferred embodiments of the present uses, the impaired bone fracture healing may be selected from the group consisting of mal-union fracture, delayed union fracture, and non-union fracture.

Provided herein is as well a method for the diagnosis, prediction, prognosis and/or monitoring of impaired fracture healing in a subject comprising measuring the level of any one or more of SDF-1, PDGF-BB, IL-8 and IL-6 in a sample from said subject. Preferably, provided herein is a method for the diagnosis, prediction, prognosis and/or monitoring of impaired bone fracture healing in a subject comprising measuring the level of IL-8 in a sample from said subject. To measure the level of one or more biomarkers, the present methods, and particularly the examination phase of such methods in which data is collected from and/or about the subject, typically comprise measuring or determining the level (i.e., quantity, amount) of said one or more biomarker(s) in a sample from the subject.

A method for the diagnosis, prediction and/or prognosis of impaired fracture healing in a subject as taught herein may in preferred embodiments comprise steps: (i) measuring the quantity of any one or more of SDF-1, PDGF-BB, IL-8 and IL-6 in a sample from the subject; (ii) comparing the quantity as measured in (i) with a reference value representing a known diagnosis, prediction and/or prognosis of impaired fracture healing; (iii) finding a deviation or no deviation of the quantity as measured in (i) from the reference value; and (iv) attributing said finding of deviation or no deviation to a particular diagnosis, prediction and/or prognosis of impaired fracture healing in the subject. Preferably, a method as taught herein for the diagnosis, prediction and/or prognosis of impaired bone fracture healing in a subject may comprise steps: (i) measuring the quantity of IL-8 in a sample from the subject; (ii) comparing the quantity as measured in (i) with a reference value representing a known diagnosis, prediction and/or prognosis of impaired bone fracture healing; (iii) finding a deviation or no deviation of the quantity as measured in (i) from the reference value; and (iv) attributing said finding of deviation or no deviation to a particular diagnosis, prediction and/or prognosis of impaired bone fracture healing in the subject. The method may also be performed for a subject at two or more successive time points and the respective outcomes at said successive time points may be compared, whereby the presence or absence of a change between the diagnosis, prediction and/or prognosis of impaired fracture healing at said successive time points is determined. When so applied, the method can monitor a change in the diagnosis, prediction and/or prognosis of impaired fracture healing in the subject over time.

For example, a deviation of the quantity of any one or more of SDF-1, PDGF-BB, IL-8 and IL-6, preferably of the quantity of IL-8, in a sample from a subject compared to a reference value representing the prediction or diagnosis of no impaired fracture healing or representing a good prognosis for impaired fracture healing can indicate respectively that the subject has impaired fracture healing or is at risk of having impaired fracture healing or can indicate a poor prognosis for impaired fracture healing in the subject. In another example, the absence of such deviation from the reference value representing the prediction or diagnosis of no impaired fracture healing or representing a good prognosis for impaired fracture healing can indicate respectively that the subject does not have impaired fracture healing or is not at risk of having impaired fracture healing or can indicate a good prognosis for impaired fracture healing in the subject. In yet another example, the absence of such deviation from a reference value representing the prediction or diagnosis of impaired fracture healing or representing a poor prognosis for impaired fracture healing can indicate respectively that the subject has impaired fracture healing or is at risk of developing a impaired fracture healing or can indicate a poor prognosis for impaired fracture healing in the subject. A reference value representing the prediction or diagnosis of no impaired fracture healing may for example represent a healthy state, i.e., a state without a fracture, or may represent a state with a fracture that is not impaired fracture healing. A reference value representing the prediction or diagnosis of impaired fracture healing may for example represent a disease state, i.e., a state with impaired fracture healing.

Preferably but without limitation, the inventors have realised that a reduced quantity of SDF-1, a reduced quantity of PDGF-BB, an elevated quantity of IL-8 and/or an elevated quantity of IL-6 in a sample from a subject, particularly in serum or plasma from the subject, compared to respective reference value(s) representing the prediction or diagnosis of no impaired fracture healing, more preferably representing a healthy state or normally healing fracture, or representing a good prognosis for impaired fracture healing, can indicate that the subject has impaired fracture healing or is at risk of having an impaired fracture healing or can indicate a poor prognosis for fracture healing in the subject. Preferably but without limitation, the inventors have realised that a reduced quantity of SDF-1 and/or a reduced quantity of IL-6 in a sample from a subject, particularly in supernatant of cultured osteoblastic cells or mesenchymal stem cells obtained from the subject, compared to respective reference value(s) representing the prediction or diagnosis of no impaired fracture healing, more preferably representing a healthy state or normally healing fracture, or representing a good prognosis for impaired fracture healing, can indicate that the subject has impaired fracture healing or is at risk of having an impaired fracture healing or can indicate a poor prognosis for fracture healing in the subject.

A method for monitoring impaired fracture healing or for monitoring the probability of developing impaired fracture healing in a subject as taught herein may in preferred embodiments comprise steps: (i) measuring the quantity of any one or more of SDF-1, PDGF-BB , IL-8 and IL-6 in a sample from the subject at two or more successive time points; (ii) comparing the quantity as measured in (i) between said two or more successive time points; (iii) finding a deviation or no deviation of the quantity as measured in (i) between said two or more successive time points; (iv) attributing said finding of deviation or no deviation to a change in impaired fracture healing or to a change in the probability of developing impaired fracture healing in the subject between the two or more successive time points. Preferably, a method as taught herein for monitoring impaired bone fracture healing or for monitoring the risk of developing impaired bone fracture healing in a subject may comprise steps: (i) measuring the quantity of IL-8 in a sample from the subject at two or more successive time points; (ii) comparing the quantity as measured in (i) between said two or more successive time points; (iii) finding a deviation or no deviation of the quantity as measured in (i) between said two or more successive time points; (iv) attributing said finding of deviation or no deviation to a change in impaired bone fracture healing or to a change in the risk of developing impaired bone fracture healing in the subject between the two or more successive time points.

Preferably but without limitation, the inventors have realised that a reduced quantity of SDF-1, a reduced quantity of PDGF-BB, an elevated quantity of IL-8 and/or an elevated quantity of IL-6 in a sample from a subject, preferably in serum or plasma from the subject, at a later one of said two or more successive time points compared to the respective quantity at an earlier one of said two or more successive time points, can indicate that the subject's risk of developing impaired fracture healing has increased.

Preferably but without limitation, the inventors have realised that a reduced quantity of SDF-1 and/or a reduced quantity of IL-6 in a sample from a subject, particularly in supernatant of cultured osteoblastic cells or mesenchymal stem cells obtained from the subject, at a later one of said two or more successive time points compared to the respective quantity at an earlier one of said two or more successive time points, can indicate that the subject's risk of developing impaired fracture healing has increased.

Also disclosed is a method to determine whether a subject is or is not (such as, e.g., still is, or is no longer) in need of a therapeutic or prophylactic (preventative) treatment of impaired fracture healing comprising measuring the level of any one or more of SDF-1, PDGF-BB, IL-8 and IL-6 in a sample from said subject. In preferred embodiments the method may comprise steps: (i) measuring the quantity of any one or more of SDF-1, PDGF-BB, IL-8 and IL-6 in the sample from the subject; (ii) comparing the quantity as measured in (i) with a reference value representing a known diagnosis, prediction and/or prognosis of impaired fracture healing; (iii) finding a deviation or no deviation of the quantity as measured in (i) from the reference value; (iv) inferring from said finding the presence or absence of a need for a therapeutic or prophylactic treatment of impaired fracture healing. Preferably, a method to determine whether a subject is or is not in need of a therapeutic or prophylactic treatment of impaired bone fracture healing may comprise measuring the level of IL-8 in a sample from said subject, preferably may comprise steps: (i) measuring the quantity of IL-8 in the sample from the subject; (ii) comparing the quantity as measured in (i) with a reference value representing a known diagnosis, prediction and/or prognosis of impaired bone fracture healing; (iii) finding a deviation or no deviation of the quantity as measured in (i) from the reference value; (iv) inferring from said finding the presence or absence of a need for a therapeutic or prophylactic treatment of impaired bone fracture healing. A treatment may be particularly indicated where the method allows for a conclusion that the subject has impaired fracture healing or is at risk of having impaired fracture healing or has a poor prognosis for impaired fracture healing.

In this context further disclosed is a method for determining the outcome of a therapeutic or prophylactic treatment of impaired fracture healing in a subject, comprising measuring the level of any one or more of SDF-1, PDGF-BB, IL-8 and IL-6, preferably the level of IL-8, in a sample from said subject.

In preferred embodiments of the present methods, the impaired bone fracture healing may be selected from the group consisting of mal-union fracture, delayed union fracture, and non-union fracture.

Treatments of impaired fracture healing include, *inter alia,* non-invasive treatments such as, e.g., electrical stimulation, ultrasounds or specialized braces, and invasive measures, such as, e.g., surgical removal of dead tissue, insertion of internal brace (e.g., rod, plate or screw), insertion of bone graft, injection of one or more bone morphogenetic proteins (BMPs), or amputation to prevent further injury. Preferably but without limitation, the inventors have realised that a reduced quantity of SDF-1, a reduced quantity of PDGF-BB, an elevated quantity of IL-8 and/or an elevated quantity of IL-6 in a sample from a subject, particularly in serum or plasma from the subject, compared to respective reference value(s) representing the prediction or diagnosis of no impaired fracture healing, more preferably representing a healthy state, or representing a good prognosis for impaired fracture healing, can indicate that the subject needs a therapeutic or prophylactic treatment of impaired fracture healing.

Preferably but without limitation, the inventors have realised that a reduced quantity of SDF-1 and/or a reduced quantity of IL-6 in a sample from a subject, particularly in supernatant of cultured osteoblastic cells or mesenchymal stem cells obtained from the subject, compared to respective reference value(s) representing the prediction or diagnosis of no impaired fracture healing, more preferably representing a healthy state, or representing a good prognosis for impaired fracture healing, can indicate that the subject needs a therapeutic or prophylactic treatment of impaired fracture healing.

Any one or more of SDF-1, PDGF-BB, IL-8 and/or IL-6 may display their diagnostic, predictive, prognostic and/or monitoring value in impaired bone fracture healing, pre-fracture and/or post-fracture. In non-limiting embodiments, any one or more of SDF-1, PDGF-BB, IL-8 and/or IL-6 may be particularly evaluated in subjects between the time of fracture and about 40 months post fracture, such as at about 1, 2, 3 and/or 4 weeks post-fracture, and/or at about 1 to 4 months, about 5 to 8 months, about 9 to 12 months, about 13 to 16 months, about 17 to 20 months, about 21 to 24 months, about 25 to 28 months, about 29 to 32 months, about 33 to 36 months and/or about 37 to 40 months post-fracture.

Any one of the present biomarkers, uses and methods may be preferably combined with or may complement, or may be scheduled at the same time as, one or more other diagnostic measures for impaired fraction healing, such as without limitation conventional radiological and imaging techniques including X-ray, positron emission tomography (PET) scans, computed tomography (CT) scans, magnetic resonance imaging (MRI), ultrasound imaging.

Any one or more of the herein disclosed biomarkers, uses and methods may be particularly useful in subjects known or expected to be at risk of developing impaired bone fracture healing, e.g., having one or more risk factors for impaired bone fracture healing. Without limitation risk factors associated with impaired bone fracture healing include lifestyle and health factors that may interfere with bone healing, such as *inter alia* smoking, excessive alcohol use, poor nutritional status, poor general health, fitness deficits, and diabetes; factors that may contribute to loss of bone strength, such as *inter alia* use of non-steroidal anti-inflammatory drugs (NSAID), use of immunosupressive drugs, other drugs such as anticonvulsants, and the thyroid hormone replacement, thyroxine; type and site of fracture such as fracture in a poorly vascular site, instability at the fracture site, high energy trauma, and poor condition of the soft tissues around the bone; ancestry such as individuals of European or Asian ancestry who have increased risk for osteoporosis; age such as elderly individuals who are at increased risk for poor bone healing; women who have experienced early menopause, late menarche, or the loss of their ovaries and who are at increased risk for bone weakness; *etc.*

In embodiments, any one or more of the present biomarkers, uses and methods may be complemented or combined with determination of the presence or absence and/or level of one or more risk factors for impaired bone fracture healing in the subject.

Any one of the present biomarkers, uses and methods may preferably allow for sensitivity and/or specificity (preferably, sensitivity and specificity) of at least 50%, at least 60%, at least 70% or at least 80%, e.g., ≥ 85% or ≥ 90% or ≥95%, e.g., between about 80% and 100% or between about 85% and 95%.

Any one of the present biomarkers, uses and methods may be applied to subjects who have not yet been diagnosed as having impaired bone fracture healing (for example, preventative screening), or who have been subjected to a fracture, or who have been diagnosed as having impaired bone fracture healing, or who are suspected of having impaired bone fracture healing (for example, display one or more characteristic signs and/or symptoms), or who are at risk of developing impaired bone fracture healing (for example, genetic predisposition; presence of one or more developmental, environmental or behavioural risk factors). Any one of the present biomarkers, uses and methods may also be used to detect various stages of progression or severity of impaired bone fracture healing; to detect response of fracture healing to prophylactic or therapeutic treatments or other interventions; to help the medical practitioner in deciding upon worsening, status-quo, partial recovery, or complete recovery of the subject from impaired fracture healing, resulting in either further treatment or observation or in discharge of the subject from a medical care centre. Also, any one of the present biomarkers, uses and methods may be employed for population screening, such as, e.g., screening in a general population or in a population stratified based on one or more criteria, e.g., age, ancestry, occupation, presence or absence of risk factors of impaired fracture healing, etc.

Any one of the present biomarkers, uses and methods may also benefit from being further complemented or combined with the assessment of one or more other biomarkers, signs, symptoms and/or clinical parameters relevant for impaired fracture healing. By means of example and not limitation, biomarkers potentially useful in evaluating impaired fracture healing, and whose quantity may be advantageously measured in the present uses and methods, include any one or more of transforming growth factor-beta (TGF-β, other isoforms of platelet-derived growth factor (PDGF) (other isoforms), fibroblast growth factor (FGF) and bone morphogenetic protein (BMP), the levels of which have been reported decreased in impaired fracture healing patients (Brownlow et al. 2001; Weiss S et al. 2009; Zimmermann et al. 2005; all *sura*).

Hence, provided in certain embodiments are the uses and methods as defined herein, further comprising measuring the level of any one or more of FGF-β, PDGF, FGF and BMP in a sample from the subject.

Also intended in certain embodiments, are the uses and methods as defined herein, further comprising measuring the level of any one or more of SDF-1, PDGF-BB, IL-6, FGF-β, PDGF, FGF and BMP in a sample from the subject.

The respective quantity of any one or more of the present biomarkers may be evaluated separately and individually, i.e., each quantity compared with its corresponding reference value. Otherwise, the quantities of any two or more of the present biomarkers may be used to establish a biomarker profile, which can be suitably compared with a corresponding multi-parameter reference value. Alternatively, the quantities of any two or more biomarkers may each be modulated by an appropriate weighing factor and added up to yield a single value, which can then be suitably compared with a corresponding reference value obtained accordingly. One shall appreciate that such weighing factors may depend on the methodology used to quantify the biomarkers, and for each particular experimental setting may be determined and comprised in a model suitable for diagnosis, prediction and/or prognosis of impaired fracture healing.

Reference values as employed herein may be established according to known procedures previously employed for other biomarkers. Reference values may be established either within (*i.e.,* constituting a step of) or external to (*i.e.,* not constituting a step of) the uses and methods taught herein. Accordingly, any one of the uses or methods taught herein may comprise a step of establishing a requisite reference value.

Hence, also provided is a method for establishing a reference value for any one or more biomarkers as taught herein, said reference value representing:
(a) a prediction or diagnosis of the absence of impaired fracture healing or a good prognosis for impaired fracture healing, or
(b) a prediction or diagnosis of impaired fracture healing or a poor prognosis for impaired fracture healing,
comprising:
(i) measuring the quantity of the one or more biomarkers in a sample from:
   (i a) one or more subjects not having impaired fracture healing or not being at risk of having impaired fracture healing or having a good prognosis for impaired fracture healing, or
   (i b) one or more subjects having impaired fracture healing or being at risk of having impaired fracture healing or having a poor prognosis for impaired fracture healing, and
(ii a) establishing from the quantity of the one or more biomarkers as measured in (i a) the reference value representing the prediction or diagnosis of the absence of impaired fracture healing or representing the good prognosis for impaired fracture healing, or
(ii b) establishing from the quantity of the one or more biomarkers as measured in (i b) the reference value representing the prediction or diagnosis of impaired fracture healing or representing the poor prognosis for impaired fracture healing.

Preferably, also provided herein is a method for establishing a reference value for IL-8, said reference value representing:
(a) a prediction or diagnosis of the absence of impaired bone fracture healing or a good prognosis for impaired bone fracture healing, or
(b) a prediction or diagnosis of impaired bone fracture healing or a poor prognosis for impaired bone fracture healing,
comprising:
(i) measuring the quantity of IL-8 in a sample from:
   (i a) one or more subjects not having impaired bone fracture healing or not being at risk of having impaired bone fracture healing or having a good prognosis for impaired bone fracture healing, or
   (i b) one or more subjects having impaired bone fracture healing or being at risk of having impaired bone fracture healing or having a poor prognosis for impaired bone fracture healing, and
(ii a) establishing from the quantity of IL-8 as measured in (i a) the reference value representing the prediction or diagnosis of the absence of impaired bone fracture healing or representing the good prognosis for impaired bone fracture healing, or
(ii b) establishing from the quantity of IL-8 as measured in (i b) the reference value representing the prediction or diagnosis of impaired bone fracture healing or representing the poor prognosis for impaired bone fracture healing.

In preferred embodiments, the present uses and methods may measure the systemic quantity of the one or more biomarkers as taught herein. Preferably, the present uses and methods may comprise measuring the systemic quantity of IL-8. Systemic quantity of the one or more biomarkers, preferably Il-8, may be suitably evaluated in samples which comprise, consist essentially of or consist of whole blood or a fractional component thereof, such as preferably plasma or serum.

In other embodiments, the present uses and methods may measure the local quantity of the one or more biomarkers as taught herein at the site of the impaired healing fracture. Preferably, the present uses and methods may measure the local quantity of IL-8 at the site of the impaired healing fracture. Local quantity of the one or more biomarkers, preferably IL-8, may be suitably determined in samples which comprise, consist essentially of or consist of tissue removed from the site of the impaired healing fracture, such as tissue obtained by biopsy or other tissue recovery techniques.

In other embodiments, the present uses and methods may measure the bone marrow quantity of the one or more biomarkers as taught herein at different site of bone marrow harvesting. Preferably, the present uses and methods may measure the bone marrow quantity of IL-8 at different sites of bone marrow harvesting. The bone marrow may be obtained from the bone having the impaired healing fracture in the subject, or from a site distant from said impaired healing fracture.

In yet further embodiments, the present uses and methods may measure the quantity of the one or more biomarkers as taught herein in cells or in the supernatant of cells obtained from the subject and subsequently cultured *in vitro.* Preferably, the present uses and methods may comprise measuring the quantity of IL-8 in cells or in the supernatant of cells obtained from the subject and subsequently cultured *in vitro.* Preferably, the cells may be bone tissue cells or progenitors, more preferably osteoblasts (OB) or mesenchymal stem cells (MSC). Preferably, the biomarkers may be measured in primary and/or further (e.g., secondary, tertiary, etc.) cultures of the cells. The cells may be obtained from the site of the impaired healing fracture in the subject, or from a site distant from said impaired healing fracture.

Further disclosed is a kit, particularly a kit for the diagnosis, prediction, prognosis and/or monitoring of impaired fracture healing in a subject, the kit comprising (i) means for measuring the quantity of any one or more of SDF-1, PDGF-BB, IL-8 and/or IL-6, particularly in a sample from the subject, and (ii) optionally and preferably one or more reference values or means for establishing said one or more reference values, wherein said one or more reference values represent a known diagnosis, prediction and/or prognosis of impaired fracture healing.

The means for measuring the quantity of a biomarker may comprise one or more binding agents capable of specifically binding to said biomarker. Exemplary binding agents may include hybridisation (oligonucleotide) probes, amplification primers, antibodies, aptamers, photoaptamers, proteins, peptides, peptidomimetics or small molecules. Binding agents may be advantageously immobilised on a solid phase or support.

Disclosed is thus also a kit, particularly a kit for the diagnosis, prediction, prognosis and/or monitoring of impaired fracture healing in a subject, the kit comprising (i) one or more binding agents capable of specifically binding to any one or more of SDF-1, PDGF-BB , IL-8 and/or IL-6 , particularly in a sample from the subject, (ii) preferably, a known quantity or concentration of said any one or more of SDF-1, PDGF-BB , IL-8 and/or IL-6 , such as for use as controls, standards and/or calibrators, (iii) optionally and preferably, one or more reference values or means for establishing said one or more reference values, wherein said one or more reference values represent a known diagnosis, prediction and/or prognosis of impaired fracture healing. Said components under (i) and/or (ii) may be suitably labelled as taught elsewhere in this specification.

In preferred embodiments, the kits may be configured as portable devices, such as, for example, bedside devices, for use at home or in clinical settings.

It shall be appreciated that means or tools for collecting a sample from a subject, such as, e.g., a conventional blood collection tube comprising an anti-clotting agent, may be provided separately from or may be comprised in the kits disclosed herein.

Further disclosed is the use of any one kit as described herein for the diagnosis, prediction, prognosis and/or monitoring of impaired fracture healing.

Preferably, provided is use of a kit for the diagnosis, prediction, prognosis and/or monitoring of impaired bone fracture healing in a subject, the kit comprising (i) means for measuring the quantity of IL-8, particularly in a sample from the subject, and (ii) optionally and preferably one or more reference values or means for establishing said one or more reference values, wherein said one or more reference values represent a known diagnosis, prediction and/or prognosis of impaired bone fracture healing. Further provided is the use as taught herein, wherein means for collecting a sample from a subject is provided separately from or is comprised in the kit.

Also disclosed are reagents and tools useful for measuring any one or more biomarkers as taught herein. Hence, disclosed is a nucleic acid array or microarray or a protein, polypeptide or peptide array or microarray comprising any one, preferably any two, preferably any three, more preferably all four of SDF-1, PDGF-BB , IL-8 and/or IL-6 . Also disclosed is a binding agent array or microarray comprising one or more binding agents capable of specifically binding to any one, preferably any two, preferably three, more preferably all four of SDF-1, PDGF-BB , IL-8 and/or IL-6 , preferably comprising a known quantity or concentration of said one or more binding agents.

Further disclosed is the use of any one array or microarray as described herein for the diagnosis, prediction, prognosis and/or monitoring of impaired fracture healing.

Preferably, provided is use of a nucleic acid array or microarray or a protein, polypeptide or peptide array or microarray for the diagnosis, prediction, prognosis and/or monitoring of impaired bone fracture healing, said array or microarray comprising a nucleic acid encoding IL-8 or comprising IL-8. Preferably, also provided is use of a binding agent array or microarray for the diagnosis, prediction, prognosis and/or monitoring of impaired bone fracture healing, said array or microarray comprising one or more binding agents capable of specifically binding to IL-8, preferably comprising a known quantity or concentration of said one or more binding agents.

The above and further aspects and preferred embodiments of the invention are described in the following sections and in the appended claims. The subject matter of appended claims is hereby specifically incorporated in this specification.

### BRIEF DESCRIPTION OF FIGURES

**Figure 1** illustrates plasma levels of SDF-1 in an experiment comparing a group of non-union patients (NU) with healthy controls (HV), (A) all samples (HV, n = 49; NU, n = 15), (B) samples where plasma is collected in heparin tubes (HV, n = 26; NU, n = 11), (C) samples where plasma is collected in EDTA tubes (HV, n = 40; NU, n = 5).
**Figure 2** illustrates serum levels of PDGF-BB in an experiment comparing a group of non-union patients (NU, n = 9) with healthy controls (HV, n = 20).
**Figure 3** illustrates serum levels of IL-8 in an experiment comparing a group of non-union patients (NU, n = 4) with healthy controls (HV, n = 18).
**Figure 4** illustrates serum levels of IL-6 in an experiment comparing a group of non-union patients (NU; n = 13) with healthy controls (HV; n = 29).
**Figure 5A** illustrates levels of SDF-1 in supernatant of osteoblastic cell (OB) culture comparing a group of non-union patients (NU, n = 6) with healthy controls (HV, n = 9).
**Figure 5B** illustrates levels of SDF-1 in supernatant of mesenchymal cell (MSC) culture comparing a group of non-union patients (NU, n = 6) with healthy controls (HV, n = 9).
**Figure 6** illustrates levels of IL-6 in supernatant of osteoblastic cell (OB) culture comparing a group of non-union patients (NU, n = 6) with healthy controls (HV, n = 10).

### DETAILED DESCRIPTION OF THE INVENTION

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. The term also encompasses "consisting of" and "consisting essentially of".

The recitation of numerical ranges by endpoints includes all numbers and fractions subsumed within the respective ranges, as well as the recited endpoints.

The term "about" as used herein when referring to a measurable value such as a parameter, an amount, a temporal duration, and the like, is meant to encompass variations of and from the specified value, in particular variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" refers is itself also specifically, and preferably, disclosed.

Whereas the term "one or more", such as one or more members of a group of members, is clear per se, by means of further exemplification, the term encompasses *inter alia* a reference to any one of said members, or to any two or more of said members, such as, e.g., any ≥3, ≥4, ≥5, ≥6 or ≥7 etc. of said members, and up to all said members.

Unless otherwise specified, all terms used in disclosing the invention, including technical and scientific terms, have the meaning as commonly understood by one of ordinary skill in the art to which this invention belongs. By means of further guidance, term definitions may be included to better appreciate the teaching of the present invention.

The inventors identified stromal derived factor-1 (SDF-1), platelet-derived growth factor BB (PDGF-BB), interleukin-8 (IL-8) and interleukin 6 (IL-6) as novel, useful biomarker(s) for the diagnosis, prediction, prognosis and/or monitoring of impaired bone fracture healing. In particular, the inventors identified IL-8 as novel, useful biomarker for the diagnosis, prediction, prognosis and/or monitoring of impaired bone fracture healing.

The term "biomarker" is widespread in the art and may broadly denote a biological molecule and/or a detectable portion thereof whose qualitative and/or quantitative evaluation in a subject is, alone or combined with other data, predictive and/or informative (e.g., predictive, diagnostic and/or prognostic) with respect to one or more aspects of the subject's phenotype and/or genotype, such as, for example, with respect to the status of the subject as to a given disease or condition. Particularly, biomarkers as intended herein may be RNA-based (esp. mRNA-based), or preferably may be protein-, polypeptide-or peptide-based.

The terms "non-union fracture", "fracture non-union", "non-union" or "NU" interchangeably concern a fracture which due to various factors fails to heal in a normal time period. NU includes *inter alia* tight non-unions and unstable non-unions or pseudarthrosis. The terms "mal-union fracture", "fracture mal-union" or "mal-union" interchangeably concern an imperfect union of previously fragmented bone. The terms "delayed union fracture" or "delayed union" interchangeably relate to a fracture in which healing has not occurred in the expected time and the outcome remains uncertain. Non-union, mal-union and delayed union fractures are encompassed herein by the term "impaired bone fracture healing" or "impaired fracture healing". Impaired fracture healing hence requires some form of intervention to stimulate healing.

The time period at which impaired fracture healing is concluded in practice varies depending on the particular fracture, but it is generally accepted that a fracture not healed by 6 months post injury will not heal without intervention. It has also been suggested to conclude that impaired fracture healing will result if a fracture shows no sign of progressing towards healing by 3 months post injury, or simply if a fracture has not healed in the time an experienced fracture surgeon would expect it to heal.

Reference throughout this specification to diseases or conditions encompasses any such diseases or conditions as disclosed herein insofar consistent with the context of a particular recitation, more specifically encompasses impaired fracture healing.

The terms "predicting" or "prediction", "diagnosing" or "diagnosis" and "prognosticating" or "prognosis" are commonplace and well-understood in medical and clinical practice. It shall be understood that the phrase "a method for the diagnosis, prediction and/or prognosis" a given disease or condition may also be interchanged with phrases such as "a method for diagnosing, predicting and/or prognosticating" of said disease or condition or "a method for making (or determining or establishing) the diagnosis, prediction and/or prognosis" of said disease or condition, or the like.

By means of further explanation and without limitation, "predicting" or "prediction" generally refer to an advance declaration, indication or foretelling of a disease or condition in a subject not (yet) having said disease or condition. For example, a prediction of a disease or condition in a subject may indicate a probability, chance or risk that the subject will develop said disease or condition, for example within a certain time period or by a certain age. Said probability, chance or risk may be indicated *inter alia* as an absolute value, range or statistics, or may be indicated relative to a suitable control subject or subject population (such as, *e.g.,* relative to a general, normal or healthy subject or subject population). Hence, the probability, chance or risk that a subject will develop a disease or condition may be advantageously indicated as increased or decreased, or as fold-increased or fold-decreased relative to a suitable control subject or subject population. As used herein, the term "prediction" of the conditions or diseases as taught herein in a subject may also particularly mean that the subject has a 'positive' prediction of such, *i.e.,* that the subject is at risk of having such (*e.g.,* the risk is significantly increased vis-a-vis a control subject or subject population). The term "prediction of no" diseases or conditions as taught herein as described herein in a subject may particularly mean that the subject has a 'negative' prediction of such, *i.e.,* that the subject's risk of having such is not significantly increased vis-à-vis a control subject or subject population.

The terms "diagnosing" or "diagnosis" generally refer to the process or act of recognising, deciding on or concluding on a disease or condition in a subject on the basis of symptoms and signs and/or from results of various diagnostic procedures (such as, for example, from knowing the presence, absence and/or quantity of one or more biomarkers characteristic of the diagnosed disease or condition). As used herein, "diagnosis of" the diseases or conditions as taught herein in a subject may particularly mean that the subject has such, hence, is diagnosed as having such. "Diagnosis of no" diseases or conditions as taught herein in a subject may particularly mean that the subject does not have such, hence, is diagnosed as not having such. A subject may be diagnosed as not having such despite displaying one or more conventional symptoms or signs reminiscent of such.

The terms "prognosticating" or "prognosis" generally refer to an anticipation on the progression of a disease or condition and the prospect (*e.g.,* the probability, duration, and/or extent) of recovery. A good prognosis of the diseases or conditions taught herein may generally encompass anticipation of a satisfactory partial or complete recovery from the diseases or conditions, preferably within an acceptable time period. A good prognosis of such may more commonly encompass anticipation of not further worsening or aggravating of such, preferably within a given time period. A poor prognosis of the diseases or conditions as taught herein may generally encompass anticipation of a substandard recovery and/or unsatisfactorily slow recovery, or to substantially no recovery or even further worsening of such.

Hence, prediction or prognosis of a disease or condition can *inter alia* allow to predict or make a prognosis of the occurrence of the disease or condition, or to predict or make a prognosis of the progression, aggravation, alleviation or recurrence of the disease or condition or response to treatment or to other external or internal factors, situations or stressors, *etc.*

Further, monitoring a disease or condition can *inter alia* allow to predict the occurrence of the disease or condition, or to monitor the progression, aggravation, alleviation or recurrence of the disease or condition, or response to treatment or to other external or internal factors, situations or stressors, *etc.* Advantageously, monitoring may be applied in the course of a medical treatment of a subject, preferably medical treatment aimed at alleviating the so-monitored disease or condition. Such monitoring may be comprised, *e.g.,* in decision making whether a patient may be discharged, needs a change in treatment or needs further hospitalisation. As intended herein, a reference to monitoring of a disease or condition also specifically includes monitoring of the probability, risk or chance of a subject to develop the disease or condition, i.e., monitoring change(s) in said probability, risk or chance over time.

The term "subject" or "patient" as used herein typically and preferably denotes humans, but may also encompass reference to non-human animals, preferably warm-blooded animals, more preferably vertebrates, even more preferably mammals, such as, e.g., non-human primates, rodents, canines, felines, equines, ovines, porcines, and the like. Particularly intended are subjects known or suspected to have suffered a bone fracture, more particularly wherein the bone fracture has not healed (as established, e.g., by radiological investigation). Suitable subjects may include ones presenting to a physician with symptoms and signs indicative of an unhealed bone fracture or impaired healing of bone fracture.

The terms "sample" or "biological sample" as used herein include any biological specimen obtained from a subject. Exemplary biological specimens include, without limitation, whole blood, plasma, serum, red blood cells, white blood cells (e.g., peripheral blood mononuclear cells), whole bone marrow, bone marrow-derived serum and stem cells, saliva, urine, stool (i.e., faeces), tears, sweat, sebum, nipple aspirate, ductal lavage, tumour exudates, synovial fluid, cerebrospinal fluid, lymph, fine needle aspirate, amniotic fluid, any other bodily fluid, nail clippings, cell lysates, cellular secretion products, inflammation fluid, vaginal secretions, biopsies, bone biopsies, bone tissues, tissue homogenates, etc. Preferred samples may include ones comprising any one or more biomarkers as taught herein in detectable quantities. More preferred samples, particularly for determination of systemic levels of biomarkers, include whole blood or a fractional component thereof, such as particularly preferably plasma or serum. In some embodiments, a sample may comprise or may be represented by cells or cell supernatant, said cells (preferably MSC or OB) having been obtained from the subject and subsequently cultured *in vitro.* Preferably, the cells may be evaluated during primary and/or secondary culture. Preferably a sample is readily obtainable by minimally invasive methods, allowing to remove or isolate said sample from the subject.

A molecule or analyte such as a metabolite, nucleic acid, RNA, DNA or cDNA, protein, polypeptide or peptide, is "measured" in a sample when the presence or absence and/or quantity of said molecule or analyte or of said group of molecules or analytes is detected or determined in the sample, preferably substantially to the exclusion of other molecules and analytes. For example, a biomarker may be measured by measuring the mRNA encoding the same, or by measuring the encoded protein or polypeptide or a peptide thereof.

The terms "quantity", "amount" and "level" are synonymous and generally well-understood in the art. With respect to molecules or analytes, the terms may particularly refer to an absolute quantification of the molecule or analyte in a sample, or to a relative quantification of the molecule or analyte in the sample, *i.e.,* relative to another value such as relative to a reference value as taught herein, or to a range of values indicating a base-line expression of the biomarker. These values or ranges can be obtained from a single patient or from a group of patients.

An absolute quantity of a molecule or analyte in a sample may be advantageously expressed as weight or as molar amount, or more commonly as a concentration, *e.g.* weight per volume or mol per volume.

A relative quantity of a molecule or analyte in a sample may be advantageously expressed as an increase or decrease or as a fold-increase or fold-decrease relative to said another value, such as relative to a reference value as taught herein. Performing a relative comparison between first and second variables (e.g., first and second quantities) may but need not require to first determine the absolute values of said first and second variables. For example, a measurement method can produce quantifiable readouts (such as, e.g., signal intensities) for said first and second variables, wherein said readouts are a function of the value of said variables, and wherein said readouts can be directly compared to produce a relative value for the first variable vs. the second variable, without the actual need to first convert the readouts to absolute values of the respective variables.

As used herein, the reference to any one biomarker, nucleic acid, protein, polypeptide or peptide corresponds to the biomarker, nucleic acid, protein, polypeptide or peptide commonly known under the respective designations in the art. The terms encompass such biomarkers, nucleic acids, proteins, polypeptides or peptides of any organism where found, and particularly of animals, preferably warm-blooded animals, more preferably vertebrates, yet more preferably mammals, including humans and non-human mammals, still more preferably of humans. The terms particularly encompass such biomarkers, nucleic acids, proteins, polypeptides or peptides with a native sequence, i.e., ones of which the primary sequence is the same as that of the biomarkers, nucleic acids, proteins, polypeptides or peptides found in or derived from nature. A skilled person understands that native sequences may differ between different species due to genetic divergence between such species. Moreover, native sequences may differ between or within different individuals of the same species due to normal genetic diversity (variation) within a given species. Also, native sequences may differ between or even within different individuals of the same species due to post-transcriptional or post-translational modifications. Any such variants or isoforms of biomarkers, nucleic acids, proteins, polypeptides or peptides are intended herein. Accordingly, all sequences of biomarkers, nucleic acids, proteins, polypeptides or peptides found in or derived from nature are considered "native". The terms encompass the biomarkers, nucleic acids, proteins, polypeptides or peptides when forming a part of a living organism, organ, tissue or cell, when forming a part of a biological sample, as well as when at least partly isolated from such sources. The terms also encompass the biomarkers, nucleic acids, proteins, polypeptides or peptides when produced by recombinant or synthetic means.

Exemplary human biomarkers, nucleic acids, proteins, polypeptides or peptides as taught herein may be as annotated under NCBI Genbank (http://www.ncbi.nlm.nih.gov/) accession numbers given below. A skilled person can also appreciate that in some instances said sequences may be of precursors (e.g., preproteins) of the of biomarkers, nucleic acids, proteins, polypeptides or peptides as taught herein and may include parts which are processed away from the mature biomarkers, nucleic acids, proteins, polypeptides or peptides. A skilled person can further appreciate that although only one or more isoforms may be listed below, all isoforms are intended. Unless otherwise specified, the entries below are presented in the form: Name (Code; Genbank accession number for one or more representative mRNA sequences (e.g., isoforms), followed by a period and the Genbank sequence version; Genbank accession number for one or more corresponding representative amino acid sequences (e.g., isoforms), followed by a period and the Genbank sequence version):
Stromal derived factor-1 and isoforms α, β, γ, ϕ and ε (SDF-1 or CXCL12; NM_199168.3, NM_000609.5, NM_001033886.2, NM_001178134.1; NP_954637.1, NP_000600.1, NP_001029058.1, Nip_001171605.1)
Platelet-derived growth factor BB, i.e., a homodimer of PDGFB beta polypeptides (PDGFB; NM_002608.2, NM_033016.2; NP_002599.1, NP_148937.1)
Interleukin-8 (IL-8, CXCL8, GCP-1, LECT, LUCT, LYNAP, MDNCF, MONAP, NAF, NAP-1 or NAP1; NM_000584.2; NP_000575.1)
Interleukin-6 (IL-6, HSF, HGF, CDF, BSF2 or IFNB2; NM_000600.3, Nip_000591.1)

Preferably, when the sample is whole blood or a fractional component thereof such as plasma or serum, any biomarker, nucleic acid, protein, polypeptide or peptide may be a circulating form (i.e., not cell- or membrane-bound form).

Unless otherwise apparent from the context, reference herein to any biomarker, nucleic acid, protein, polypeptide or peptide thereof may generally also encompass modified forms of said biomarker, nucleic acid, protein, polypeptide or peptide such as bearing post-expression modifications including, for example, phosphorylation, glycosylation, lipidation, methylation, cysteinylation, sulphonation, glutathionylation, acetylation, oxidation of methionine to methionine sulphoxide or methionine sulphone, and the like.

In an embodiment, any biomarker, nucleic acid, protein, polypeptide or peptide may be human, *i.e.,* their primary sequence may be the same as a corresponding primary sequence of or present in a naturally occurring human biomarker, nucleic acid, protein, polypeptide or peptide. Hence, the qualifier "human" in this connection relates to the primary sequence of the respective biomarker, nucleic acid, protein, polypeptide or peptide, rather than to its origin or source. For example, such biomarker, nucleic acid, protein, polypeptide or peptide may be present in or isolated from samples of human subjects or may be obtained by other means (*e.g.,* by recombinant expression, cell-free translation or non-biological peptide synthesis).

Preferably, biomarkers as intended herein are protein-, polypeptide- or peptide-based.

The reference herein to any biomarker, nucleic acid, protein, polypeptide or peptide may also encompass fragments thereof. Hence, the reference herein to measuring (or measuring the quantity of) any one biomarker, nucleic acid, protein, polypeptide or peptide may encompass measuring the biomarker, nucleic acid, protein, polypeptide or peptide and/or measuring one or more fragments thereof. For example, any biomarker, nucleic acid, protein, polypeptide or peptide and/or one or more fragments thereof may be measured collectively, such that the measured quantity corresponds to the sum amounts of the collectively measured species. In another example, any biomarker, nucleic acid, protein, polypeptide or peptide and/or one or more fragments thereof may be measured each individually.

The term "fragment" of a nucleic acid generally refers to 5'- and/or 3'-terminally deleted or truncated forms of said nucleic acid. The term "fragment" of a protein, polypeptide or peptide generally refers to N-terminally and/or C-terminally deleted or truncated forms of said protein, polypeptide or peptide. Without limitation, a fragment of a nucleic acid, protein, polypeptide or peptide may represent at least about 5%, or at least about 10%, e.g., ≥ 20%, ≥ 30% or ≥ 40%, such as preferably ≥ 50%, e.g., ≥ 60%, ≥ 70% or ≥ 80%, or more preferably ≥ 90% or ≥ 95% of the nucleotide sequence of said nucleic acid or of the amino acid sequence of said protein, polypeptide or peptide.

In some embodiments, biomarkers or other reagents disclosed herein may comprise a detectable label. The term "label" refers to any atom, molecule, moiety or biomolecule that can be used to provide a detectable and preferably quantifiable read-out or property, and that can be attached to or made part of an entity of interest, such as a peptide or polypeptide or a specific-binding agent. Labels may be suitably detectable by mass spectrometric, spectroscopic, optical, colourimetric, magnetic, photochemical, biochemical, immunochemical or chemical means. Labels include without limitation dyes; radiolabels such as ³²p, ³³P, ³⁵S, ¹²⁵I, ¹³¹I; electron-dense reagents; enzymes (e.g. , horse-radish peroxidase or alkaline phosphatase as commonly used in immunoassays); binding moieties such as biotin, maltose; haptens such as digoxigenin, his-tag, myc-tag; luminogenic, phosphorescent or fluorogenic moieties; mass tags; and fluorescent dyes alone or in combination with moieties that can suppress or shift emission spectra by fluorescence resonance energy transfer (FRET). Examples of associations which can be utilised in the label:binding partner arrangement may include, for example biotin:streptavidin, his-tag:metal ion (e.g., Ni²⁺), maltose:maltose binding protein.

Also contemplated herein is the use of any biomarkers, nucleic acids, proteins, polypeptides or peptides as taught herein, optionally comprising a detectable label, as (positive) controls, standards or calibators in qualitative or quantitative detection assays (measurement methods) of said biomarkers, nucleic acids, proteins, polypeptides or peptides, and particularly in such methods for the diagnosis, prediction, prognosis and/or monitoring the diseases or conditions as taught herein in subjects. The biomarkers, nucleic acids, proteins, polypeptides or peptides may be supplied in any form, *inter alia* as precipitate, vacuum-dried, lyophilisate, in solution as liquid or frozen, or covalently or non-covalently immobilised on solid phase, such as for example, on solid chromatographic matrix or on glass or plastic or other suitable surfaces (e.g., as a part of peptide arrays and microarrays). The biomarkers, nucleic acids, proteins, polypeptides or peptides may be readily prepared, for example, isolated from natural sources, or prepared recombinantly or synthetically.

Further disclosed are binding agents capable of specifically binding to biomarkers, nucleic acids, proteins, polypeptides or peptides as taught herein. Binding agents as intended throughout this specification may include *inter alia* hybridisation probes, amplification primers, antibody, aptamer, photoaptamer, protein, peptide, peptidomimetic or a small molecule. The binding agents may be suitably labelled.

The term "specifically bind" as used throughout this specification means that an agent (denoted herein also as "specific-binding agent") binds to one or more desired molecules or analytes substantially to the exclusion of other molecules which are random or unrelated, and optionally substantially to the exclusion of other molecules that are structurally related. The term "specifically bind" does not necessarily require that an agent binds exclusively to its intended target(s). For example, an agent may be said to specifically bind to target(s) of interest if its affinity for such intended target(s) under the conditions of binding is at least about 2-fold greater, preferably at least about 5-fold greater, more preferably at least about 10-fold greater, yet more preferably at least about 25-fold greater, still more preferably at least about 50-fold greater, and even more preferably at least about 100-fold or more greater, than its affinity for a non-target molecule. For hybridisation probes, specific binding may be assessed under high stringency hybridisation conditions as known in the art. For amplification primers, specific binding may be evidenced by selective amplification of the intended target.

Preferably, the agent may bind to its intended target(s) with affinity constant (K_{A}) of such binding K_{A} ≥ 1×10⁶ M⁻¹, more preferably K_{A} ≥ 1×10⁷ M⁻¹, yet more preferably K_{A} ≥ 1×10⁸ M⁻¹, even more preferably K_{A} ≥ 1×10⁹ M⁻¹, and still more preferably K_{A} ≥ 1×10¹⁰ M⁻¹ or K_{A} ≥ 1×10¹¹ M⁻¹, wherein K_{A} = [SBA_T]/[SBA][T], SBA denotes the specific-binding agent, T denotes the intended target. Determination of K_{A} can be carried out by methods known in the art, such as for example, using equilibrium dialysis and Scatchard plot analysis.

As used herein, the term "antibody" is used in its broadest sense and generally refers to any immunologic binding agent. The term specifically encompasses intact monoclonal antibodies, polyclonal antibodies, multivalent (*e.g.,* 2-, 3- or more-valent) and/or multi-specific antibodies (*e.g.,* bi-or more-specific antibodies) formed from at least two intact antibodies, and antibody fragments insofar they exhibit the desired biological activity (particularly, ability to specifically bind an antigen of interest), as well as multivalent and/or multi-specific composites of such fragments. The term "antibody" is not only inclusive of antibodies generated by methods comprising immunisation, but also includes any polypeptide, e.g., a recombinantly expressed polypeptide, which is made to encompass at least one complementarity-determining region (CDR) capable of specifically binding to an epitope on an antigen of interest. Hence, the term applies to such molecules regardless whether they are produced in vitro or in vivo.

An antibody may be any of IgA, IgD, IgE, IgG and IgM classes, and preferably IgG class antibody. An antibody may be a polyclonal antibody, e.g., an antiserum or immunoglobulins purified there from (e.g., affinity-purified). An antibody may be a monoclonal antibody or a mixture of monoclonal antibodies. Monoclonal antibodies can target a particular antigen or a particular epitope within an antigen with greater selectivity and reproducibility. By means of example and not limitation, monoclonal antibodies may be made by the hybridoma method first described by Kohler et al. 1975 (Nature 256: 495), or may be made by recombinant DNA methods (e.g., as in US 4,816,567). Monoclonal antibodies may also be isolated from phage antibody libraries using techniques as described by Clackson et al. 1991 (Nature 352: 624-628) and Marks et al. 1991 (J Mol Biol 222: 581-597), for example.

Antibody binding agents may be antibody fragments. "Antibody fragments" comprise a portion of an intact antibody, comprising the antigen-binding or variable region thereof. Examples of antibody fragments include Fab, Fab', F(ab')2, Fv and scFv fragments; diabodies; linear antibodies; single-chain antibody molecules; and multivalent and/or multispecific antibodies formed from antibody fragment(s), *e.g.,* dibodies, tribodies, and multibodies. The above designations Fab, Fab', F(ab')2, Fv, scFv *etc.* are intended to have their art-established meaning.

The term antibody includes antibodies originating from or comprising one or more portions derived from any animal species, preferably vertebrate species, including, *e.g.,* birds and mammals. Without limitation, the antibodies may be chicken, turkey, goose, duck, guinea fowl, quail or pheasant. Also without limitation, the antibodies may be human, murine (e.g., mouse, rat, etc.), donkey, rabbit, goat, sheep, guinea pig, camel (e.g., *Camelus bactrianus* and *Camelus dromaderius*)*,* lama (e.g., *Lama paccos, Lama glama* or *Lama vicugna*) or horse.

A skilled person will understand that an antibody can include one or more amino acid deletions, additions and/or substitutions (e.g., conservative substitutions), insofar such alterations preserve its binding of the respective antigen. An antibody may also include one or more native or artificial modifications of its constituent amino acid residues (e.g., glycosylation, etc.).

Methods of producing polyclonal and monoclonal antibodies as well as fragments thereof are well known in the art, as are methods to produce recombinant antibodies or fragments thereof (see for example, Harlow and Lane, "Antibodies: A Laboratory Manual", Cold Spring Harbour Laboratory, New York, 1988; Harlow and Lane, "Using Antibodies: A Laboratory Manual", Cold Spring Harbour Laboratory, New York, 1999, ISBN 0879695447; "Monoclonal Antibodies: A Manual of Techniques", by Zola, ed., CRC Press 1987, ISBN 0849364760; "Monoclonal Antibodies: A Practical Approach", by Dean & Shepherd, eds., Oxford University Press 2000, ISBN 0199637229; Methods in Molecular Biology, vol. 248: "Antibody Engineering: Methods and Protocols", Lo, ed., Humana Press 2004, ISBN 1588290921).

The term "aptamer" refers to single-stranded or double-stranded oligo-DNA, oligo-RNA or oligo-DNA/RNA or any analogue thereof, that can specifically bind to a target molecule such as a peptide. Advantageously, aptamers can display fairly high specificity and affinity (*e.g.,* K_{A} in the order 1×10⁹ M⁻¹) for their targets. Aptamer production is described *inter alia* in US 5,270,163; Ellington & Szostak 1990 (Nature 346: 818-822); Tuerk & Gold 1990 (Science 249: 505-510); or "The Aptamer Handbook: Functional Oligonucleotides and Their Applications", by Klussmann, ed., Wiley-VCH 2006, ISBN 3527310592, incorporated by reference herein. The term "photoaptamer" refers to an aptamer that contains one or more photoreactive functional groups that can covalently bind to or crosslink with a target molecule. The term "peptidomimetic" refers to a non-peptide agent that is a topological analogue of a corresponding peptide. Methods of rationally designing peptidomimetics of peptides are known in the art. For example, the rational design of three peptidomimetics based on the sulphated 8-mer peptide CCK26-33, and of two peptidomimetics based on the 11-me peptide Substance P, and related peptidomimetic design principles, are described in Horwell 1995 (Trends Biotechnol 13: 132-134).

The term "small molecule" refers to compounds, preferably organic compounds, with a size comparable to those organic molecules generally used in pharmaceuticals. The term excludes biological macromolecules (*e.g.,* proteins, nucleic acids, etc.). Preferred small organic molecules range in size up to about 5000 Da, *e.g.,* up to about 4000, preferably up to 3000 Da, more preferably up to 2000 Da, even more preferably up to about 1000 Da, *e.g.,* up to about 900, 800, 700, 600 or up to about 500 Da.

Any existing, available or conventional separation, detection and quantification methods can be used herein to measure the presence or absence (e.g., readout being present vs. absent; or detectable amount vs. undetectable amount) and/or quantity (e.g., readout being an absolute or relative quantity, such as, for example, absolute or relative concentration) of biomarkers, nucleic acids, proteins, polypeptides or peptides thereof in samples (any molecules or analytes of interest to be so-measured in samples, including any one or more biomarkers, nucleic acids, proteins, polypeptides or peptides as taught herein, may be herein below referred to collectively as biomarkers).

For example, such methods may include biochemical assay methods, immunoassay methods, mass spectrometry analysis methods, or chromatography methods, or combinations thereof.

The term "immunoassay" generally refers to methods known as such for detecting one or more molecules or analytes of interest in a sample, wherein specificity of an immunoassay for the molecule(s) or analyte(s) of interest is conferred by specific binding between a specific-binding agent, commonly an antibody, and the molecule(s) or analyte(s) of interest. Immunoassay technologies include without limitation direct ELISA (enzyme-linked immunosorbent assay), indirect ELISA, sandwich ELISA, competitive ELISA, multiplex ELISA, radioimmunoassay (RIA), ELISPOT technologies, and other similar techniques known in the art. Principles of these immunoassay methods are known in the art, for example John R. Crowther, "The ELISA Guidebook", 1st ed., Humana Press 2000, ISBN 0896037282.

By means of further explanation and not limitation, direct ELISA employs a labelled primary antibody to bind to and thereby quantify target antigen in a sample immobilised on a solid support such as a microwell plate. Indirect ELISA uses a non-labelled primary antibody which binds to the target antigen and a secondary labelled antibody that recognises and allows to quantify the antigen-bound primary antibody. In sandwich ELISA the target antigen is captured from a sample using an immobilised 'capture' antibody which binds to one antigenic site within the antigen, and subsequent to removal of non-bound analytes the so-captured antigen is detected using a 'detection' antibody which binds to another antigenic site within said antigen, where the detection antibody may be directly labelled or indirectly detectable as above. Competitive ELISA uses a labelled 'competitor' that may either be the primary antibody or the target antigen. In an example, non-labelled immobilised primary antibody is incubated with a sample, this reaction is allowed to reach equilibrium, and then labelled target antigen is added. The latter will bind to the primary antibody wherever its binding sites are not yet occupied by non-labelled target antigen from the sample. Thus, the detected amount of bound labelled antigen inversely correlates with the amount of non-labelled antigen in the sample. Multiplex ELISA allows simultaneous detection of two or more analytes within a single compartment (e.g., microplate well) usually at a plurality of array addresses (see, for example, Nielsen & Geierstanger 2004. J Immunol Methods 290: 107-20 and Ling et al. 2007. Expert Rev Mol Diagn 7: 87-98 for further guidance). As appreciated, labelling in ELISA technologies is usually by enzyme (such as, e.g., horse-radish peroxidase) conjugation and the end-point is typically colourimetric, chemiluminescent or fluorescent, magnetic, piezo electric, pyroelectric and other.

Radioimmunoassay (RIA) is a competition-based technique and involves mixing known quantities of radioactively-labelled (e.g., ¹²⁵I- or ¹³¹I-labelled) target antigen with antibody to said antigen, then adding non-labelled or 'cold' antigen from a sample and measuring the amount of labelled antigen displaced (see, e.g., "An Introduction to Radioimmunoassay and Related Techniques", by Chard T, ed., Elsevier Science 1995, ISBN 0444821198 for guidance).

Generally, any mass spectrometric (MS) techniques that can obtain precise information on the mass of peptides, and preferably also on fragmentation and/or (partial) amino acid sequence of selected peptides (*e.g.,* in tandem mass spectrometry, MS/MS; or in post source decay, TOF MS), are useful herein. Suitable peptide MS and MS/MS techniques and systems are well-known *per se* (see, *e.g.,* Methods in Molecular Biology, vol. 146: "Mass Spectrometry of Proteins and Peptides", by Chapman, ed., Humana Press 2000, ISBN 089603609x; Biemann 1990. Methods Enzymol 193: 455-79; or Methods in Enzymology, vol. 402: "Biological Mass Spectrometry", by Burlingame, ed., Academic Press 2005, ISBN 9780121828073) and may be used herein. Peptide ion fragmentation in tandem MS (MS/MS) arrangements may be achieved using manners established in the art, such as, *e.g.,* collision induced dissociation (CID). Detection and quantification of biomarkers by mass spectrometry may involve multiple reaction monitoring (MRM), such as described among others by Kuhn et al. 2004 (Proteomics 4: 1175-86). MS peptide analysis methods may be advantageously combined with upstream peptide or protein separation or fractionation methods, such as for example with chromatographic methods.

Chromatography can also be used for measuring biomarkers. As used herein, the term "chromatography" encompasses methods for separating chemical substances, referred to as such and vastly available in the art. In a preferred approach, chromatography refers to a process in which a mixture of chemical substances (analytes) carried by a moving stream of liquid or gas ("mobile phase") is separated into components as a result of differential distribution of the analytes, as they flow around or over a stationary liquid or solid phase ("stationary phase"), between said mobile phase and said stationary phase. The stationary phase may be usually a finely divided solid, a sheet of filter material, or a thin film of a liquid on the surface of a solid, or the like. Chromatography is also widely applicable for the separation of chemical compounds of biological origin, such as, *e.g.,* amino acids, proteins, fragments of proteins or peptides, *etc.*

Chromatography as used herein may be preferably columnar *(i.e.,* wherein the stationary phase is deposited or packed in a column), preferably liquid chromatography, and yet more preferably HPLC. While particulars of chromatography are well known in the art, for further guidance see, e.g., Meyer M., 1998, ISBN: 047198373X, and "Practical HPLC Methodology and Applications", Bidlingmeyer, B. A., John Wiley & Sons Inc., 1993. Exemplary types of chromatography include, without limitation, high-performance liquid chromatography (HPLC), normal phase HPLC (NP-HPLC), reversed phase HPLC (RP-HPLC), ion exchange chromatography (IEC), such as cation or anion exchange chromatography, hydrophilic interaction chromatography (HILIC), hydrophobic interaction chromatography (HIC), size exclusion chromatography (SEC) including gel filtration chromatography or gel permeation chromatography, chromatofocusing, affinity chromatography such as immunoaffinity, immobilised metal affinity chromatography, and the like.

Further peptide or polypeptide separation, identification or quantification methods may be used, optionally in conjunction with any of the above described analysis methods, for measuring biomarkers in the present disclosure. Such methods include, without limitation, chemical extraction partitioning, isoelectric focusing (IEF) including capillary isoelectric focusing (CIEF), capillary isotachophoresis (CITP), capillary electrochromatography (CEC), and the like, one-dimensional polyacrylamide gel electrophoresis (PAGE), two-dimensional polyacrylamide gel electrophoresis (2D-PAGE), capillary gel electrophoresis (CGE), capillary zone electrophoresis (CZE), micellar electrokinetic chromatography (MEKC), free flow electrophoresis (FFE), etc.

The level of biomarkers at the RNA level can be detected using standard quantitative RNA measurement tools known in the art. Non-limiting examples include hybridization-based analysis, microarray expression analysis, digital gene expression (DGE), RNA-in-situ hybridization (RISH), Northern-blot analysis and the like; PCR, RT-PCR, RT-qPCR, end-point PCR, digital PCR or the like; supported oligonucleotide detection, pyrosequencing, polony cyclic sequencing by synthesis, simultaneous bi-directional sequencing, single-molecule sequencing, single molecule real time sequencing, true single molecule sequencing, hybridization-assisted nanopore sequencing and sequencing by synthesis.

The various aspects and embodiments taught herein may further rely on comparing the quantity of biomarkers measured in samples from patients with reference values, wherein said reference values represent known predictions, diagnoses and/or prognoses of diseases or conditions as taught herein.

For example, distinct reference values may represent the prediction of a risk (*e.g.,* an abnormally elevated risk) of having a given disease or condition as taught herein *vs*. the prediction of no or normal risk of having said disease or condition. In another example, distinct reference values may represent predictions of differing degrees of risk of having such disease or condition.

In a further example, distinct reference values can represent the diagnosis of a given disease or condition as taught herein *vs*. the diagnosis of no such disease or condition (such as, *e.g.,* the diagnosis of healthy, or recovered from said disease or condition, *etc.*). In another example, distinct reference values may represent the diagnosis of such disease or condition of varying severity.

In yet another example, distinct reference values may represent a good prognosis for a given disease or condition as taught herein *vs*. a poor prognosis for said disease or condition. In a further example, distinct reference values may represent varyingly favourable or unfavourable prognoses for such disease or condition.

Such comparison may generally include any means to determine the presence or absence of at least one difference and optionally of the size of such difference between values being compared. A comparison may include a visual inspection, an arithmetical or statistical comparison of measurements. Such statistical comparisons include, but are not limited to, applying a rule.

Reference values may be established according to known procedures previously employed for other biomarkers and parameters. For example, a reference value may be established in an individual or a population of individuals characterised by a particular diagnosis, prediction and/or prognosis of said disease or condition (*i.e.,* for whom said diagnosis, prediction and/or prognosis of the disease or condition holds true). Such population may comprise without limitations ≥ 2, ≥ 100, or even several hundreds or more individuals.

A "deviation" of a first value from a second value may generally encompass any direction (*e.g.,* increase: first value > second value; or decrease: first value < second value) and any extent of alteration.

For example, a deviation may encompass a decrease in a first value by, without limitation, at least about 10% (about 0.9-fold or less), or by at least about 20% (about 0.8-fold or less), or by at least about 30% (about 0.7-fold or less), or by at least about 40% (about 0.6-fold or less), or by at least about 50% (about 0.5-fold or less), or by at least about 60% (about 0.4-fold or less), or by at least about 70% (about 0.3-fold or less), or by at least about 80% (about 0.2-fold or less), or by at least about 90% (about 0.1-fold or less), relative to a second value with which a comparison is being made.

For example, a deviation may encompass an increase of a first value by, without limitation, at least about 10% (about 1.1-fold or more), or by at least about 20% (about 1.2-fold or more), or by at least about 30% (about 1.3-fold or more), or by at least about 40% (about 1.4-fold or more), or by at least about 50% (about 1.5-fold or more), or by at least about 60% (about 1.6-fold or more), or by at least about 70% (about 1.7-fold or more), or by at least about 80% (about 1.8-fold or more), or by at least about 90% (about 1.9-fold or more), or by at least about 100% (about 2-fold or more), or by at least about 150% (about 2.5-fold or more), or by at least about 200% (about 3-fold or more), or by at least about 500% (about 6-fold or more), or by at least about 700% (about 8-fold or more), or like, relative to a second value with which a comparison is being made.

Preferably, a deviation may refer to a statistically significant observed alteration. For example, a deviation may refer to an observed alteration which falls outside of error margins of reference values in a given population (as expressed, for example, by standard deviation or standard error, or by a predetermined multiple thereof, e.g., ±1xSD or ±2xSD, or ±1xSE or ±2xSE). Deviation may also refer to a value falling outside of a reference range defined by values in a given population (for example, outside of a range which comprises ≥40%, ≥ 50%, ≥60%, ≥70%, ≥75% or ≥80% or ≥85% or ≥90% or ≥95% or even ≥100% of values in said population).

In a further embodiment, a deviation may be concluded if an observed alteration is beyond a given threshold or cut-off. Such threshold or cut-off may be selected as generally known in the art to provide for a chosen sensitivity and/or specificity of the diagnosis, prediction and/or prognosis methods, e.g., sensitivity and/or specificity of at least 50%, or at least 60%, or at least 70%, or at least 80%, or at least 85%, or at least 90%, or at least 95%.

It is thus made apparent that there have been provided in accordance with the invention, biomarkers, uses and methods that provide for substantial advantages in the diagnosis, prediction, prognosis and/or monitoring of impaired fracture healing. While the invention has been described in conjunction with specific embodiments thereof, it is evident that many alternatives, modifications, and variations will be apparent to those skilled in the art in light of the foregoing description. Accordingly, it is intended to embrace all such alternatives, modifications, and variations as follows in the spirit and broad scope of the appended claims.

The aspects and embodiments of the invention are further supported by the following non-limiting examples.

### EXAMPLES

### Example 1: Measurement of biomarker levels in serum/plasma

Two groups of subjects entered the study: (1) healthy volunteers (HV), (2) patients with impaired fracture healing, in particular with non-union fractures (NU). The patient population was distributed as follows:

| | **Healthy volunteer (HV)** | **Non-union (NU)** | **P** |
|---|---|---|---|
| Number of subjects | 79 | 20 | |
| Mean age (years ± SD) | 32 ± 10 | 43 ± 16 | 0.012 |
| Sex (%) Female | 67 | 20 | |
| Fracture site | - | Long bones | |
| Delay (months ± SD) | - | 25 ± 15 | |

The mean age in the two groups varied between thirty and forty years old. Non-union patients were older (P = 0.012) and were mostly male. However, the results stayed unchanged independent of gender and age. The bone sites were in long bones (radius, humerus, fibula, tibia and cubitus) except 2 fractures of the metatarsus and 2 fractures of the calcaneum. The delay between the fracture and sample harvesting varied around 25 months with a standard deviation of 15 months.

To identify the systemic biological markers of non-unions, sera were collected in dry tubes and plasma were collected in heparin or EDTA tubes, centrifuged, aliquoted and frozen at -20°C until use. These were used to determine the level of growth factors and proteins using enzyme-linked immunosorbent assays (ELISA).

Stromal-derived factor one was measured in the plasma (SDF-1/CXCL12, Duoset, R&D Systems, Abingdon, United Kingdom). The following biomarkers were measured in the serum: platelet-derived growth factor-BB (PDGF-BB, Quantikine™, R&D Systems, Abingdon, United Kingdom), interleukin-8 (IL8/CXCL8, Quantikine™, R&D Systems, Abingdon, United Kingdom) and interleukin 6 (IL-6, Quantikine™, R&D Systems, Abingdon, United Kingdom).

All continuous values are expressed as medians ± standard error of the mean (SEM), all reported P values are 1-sided, and statistical significance is assessed at the 10% level. The normality of distribution was tested with a Kolmogorov-Smirnov test. When the Kolmogorov-Smirnov test failed, differences between groups were analyzed by a Mann-Whitney test.

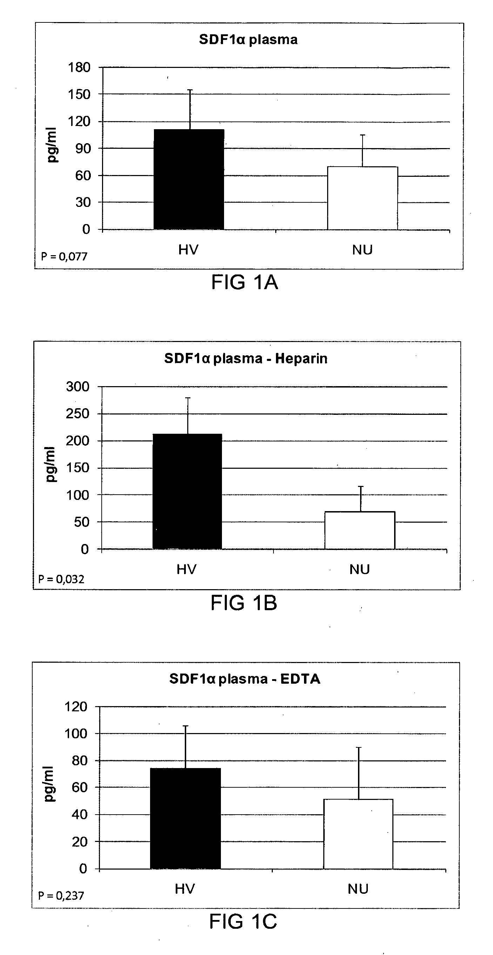

When compared to HV, in NU patients, the plasma level of SDF-1 was decreased (Fig. 1A). The decrease was more pronounced in plasma collected in heparin tubes compared with plasma collected in EDTA tubes (Fig. 1B and 1C). Hence, heparin may be a preferred blood collection agent, specifically anti-clotting agent, for the collection of blood or plasma samples for the measurement of SDF-1. Based on perusal of this particular example, and by means of illustration only and without any limitation to the invention as broadly disclosed herein, a quantity of SDF-1 in plasma of a human subject collected using heparin, which quantity is less than about 200 pg/ml, preferably less than about 150 pg/ml, more preferably less than about 100 pg/ml, such as between about 20 and about 150 pg/ml or between about 50 and about 100 pg/ml may indicate that the subject has impaired fracture healing or is at risk of having impaired fracture healing or can indicate a poor prognosis for impaired fracture healing in the subject, and may indicate that the subject needs a therapeutic or prophylactic treatment of impaired fracture healing; and a quantity of SDF-1 in plasma of a human subject collected using heparin, which quantity is more than about 200 pg/ml may indicate that the subject does not have impaired fracture healing or is not at risk of having impaired fracture healing or can indicate a good prognosis for impaired fracture healing in the subject, and may indicate that the subject does not need a therapeutic or prophylactic treatment of impaired fracture healing.

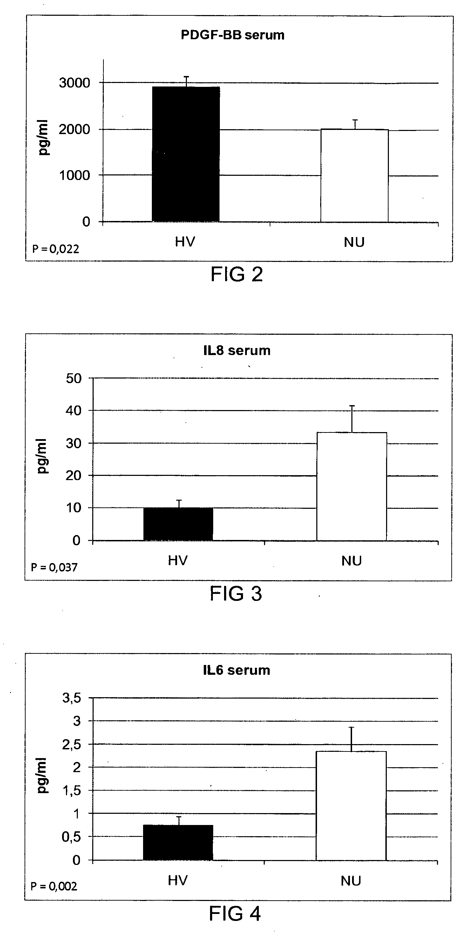

The serum level of PDGF-BB was decreased in NU patients when compared with healthy controls (HV) (Fig. 2). Based on perusal of this particular example, and by means of illustration only and without any limitation to the invention as broadly disclosed herein, a quantity of PDGF-BB in serum of a human subject which is less than about 2.50 ng/ml, preferably less than about 2.25 ng/ml, more preferably is about 2.00 ng/ml or less, such as between about 1.5 and about 2.5 ng/ml or between about 1.75 and about 2.25 ng/ml may indicate that the subject has impaired fracture healing or is at risk of having impaired fracture healing or can indicate a poor prognosis for impaired fracture healing in the subject, and may indicate that the subject needs a therapeutic or prophylactic treatment of impaired fracture healing; and a quantity of PDGF-BB in serum of a human subject which is more than about 2.50 ng/ml, preferably more than about 2.70 ng/ml, such as between about 2.50 and about 3.00 ng/ml may indicate that the subject does not have impaired fracture healing or is not at risk of having impaired fracture healing or can indicate a good prognosis for impaired fracture healing in the subject, and may indicate that the subject does not need a therapeutic or prophylactic treatment of impaired fracture healing.

When compared with healthy volunteers (HV), the serum level of IL-8 was increased in NU patients (Fig. 3). Based on perusal of this particular example, and by means of illustration only and without any limitation to the invention as broadly disclosed herein, a quantity of IL-8 in serum of a human subject which is more than about 15 pg/ml, preferably more than about 20 pg/ml, more preferably more than about 25 pg/ml, even more preferably more than about 30 pg/ml, such as between about 15 and about 45 pg/ml or between about 20 and about 40 pg/ml or between about 30 and about 35 pg/ml can indicate that the subject has impaired fracture healing or is at risk of having impaired fracture healing or can indicate a poor prognosis for impaired fracture healing in the subject, and may indicate that the subject needs a therapeutic or prophylactic treatment of impaired fracture healing; and a quantity of IL-8 in serum of a human subject which is less than about 15 pg/ml, preferably less than about 12.5 pg/ml , such as between about 5 and about 15 pg/ml or between about 7.5 and about 12.5 pg/ml or is about 10 pg/ml may indicate that the subject does not have impaired fracture healing or is not at risk of having impaired fracture healing or can indicate a good prognosis for impaired fracture healing in the subject, and may indicate that the subject does not need a therapeutic or prophylactic treatment of impaired fracture healing.

When compared with healthy volunteers (HV), the serum level of IL-6 tended to be increased in NU patients (Fig.4A and 4B). Based on perusal of this particular example, and by means of illustration only and without any limitation to the invention as broadly disclosed herein, a quantity of IL-6 in serum of a human subject which is more than about 1.0 pg/ml, preferably more than about 1.2 pg/ml, more preferably more than about 1.5 pg/ml, even more preferably more than about 2 pg/ml, such as between about 1.3 and about 2.5 pg/ml or between about 1.5 and about 2.5 pg/ml may indicate that the subject has impaired fracture healing or is at risk of having impaired fracture healing or can indicate a poor prognosis for impaired fracture healing in the subject, and may indicate that the subject needs a therapeutic or prophylactic treatment of impaired fracture healing; and a quantity of IL-6 in serum of a human subject which is less than about 1.0 pg/ml may indicate that the subject does not have impaired fracture healing or is not at risk of having impaired fracture healing or can indicate a good prognosis for impaired fracture healing in the subject, and may indicate that the subject does not need a therapeutic or prophylactic treatment of impaired fracture healing.

### Example 2: Culturing cells from subjects

As noted, the quantity of biomarkers may also be measured in cells or in the supernatant of cells obtained from subjects and cultured *in vitro,* preferably from osteoblastic cells (OB) or mesenchymal stem cells (MSC). The following provides suitable protocols for isolation, differentiation and culture of such cells.

Twenty to sixty ml of heparinised bone marrow (BM) was obtained from iliac crest distant from the fracture site. BM was mixed with phosphate-buffered saline (PBS:BM ratio (v:v): 2) and layered on density gradient Ficoll solution. After centrifugation, mononuclear cells were harvested from the interface and washed twice in PBS. The cells were plated at 1.43 x10⁶ cells/25 cm² flasks in two different media; (1) a mesenchymal medium composed of DMEM, 10% foetal bovine serum, 1% L-glutamine, 1% penicillin and 1% streptomycin; (2) an osteogenic medium. Cells were maintained in a 37°C humidified atmosphere containing 5% CO₂. Medium changes were done every 2 to 3 days. When confluent, cells of the primary culture were detached and replated for the secondary culture. The supernatants of these 2 culture passages were collected and frozen until use.

The ELISA reagents protocols used for blood samples are applied to the cell supernatants with routine adaptation.

### Example 3: Autocrine/paracrine activity of osteoblastic cells and mesenchymal stem cells

To study the autocrine/paracrine activity of osteoprogenitor cells in impaired bone fracture healing, the level of growth factors secreted in supernatant osteoblastic cell (OB) or mesenchymal cell (MSC) culture was assessed by ELISA. The following growth factors were measured; stromal-derived factor one (SDF-1/CXCL12, Duoset, R&D Systems, Abingdon, United Kingdom), and interleukin-6 (IL-6, Duoset, R&D Systems, Abingdon, United Kingdom). Values were expressed in pg/ml of supernatant.

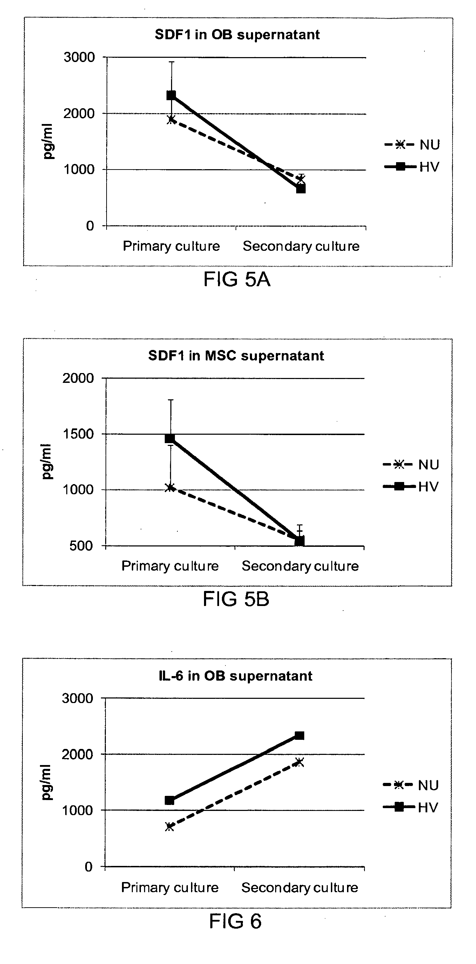

When compared with healthy volunteers (HV), SDF-1 was less secreted in supernatant of OB and MSC culture of non-union patients (NU) at the end of primary cell culture (Fig 5A and 5B).

Furthermore, IL-6 was less secreted in supernatant of OB culture of NU patients at the end of primary and secondary cell cultures when compared with HV (Fig 6).

## Claims

1. Use of interleukin-8 (IL-8) as a biomarker for impaired bone fracture healing.

2. Use of IL-8 for the diagnosis, prediction, prognosis and/or monitoring of impaired bone fracture healing.

3. The use according to any one of claims 1 or 2, wherein the impaired bone fracture healing is selected from the group consisting of mal-union fracture, delayed union fracture, and non-union fracture.

4. A method for the diagnosis, prediction, prognosis and/or monitoring of impaired bone fracture healing in a subject comprising measuring the level of IL-8 in a sample from said subject.

5. The method for the diagnosis, prediction and/or prognosis of impaired bone fracture healing in a subject according to claim 4 comprising steps: (i) measuring the quantity of IL-8 in a sample from the subject; (ii) comparing the quantity as measured in (i) with a reference value representing a known diagnosis, prediction and/or prognosis of impaired bone fracture healing; (iii) finding a deviation or no deviation of the quantity as measured in (i) from the reference value; and (iv) attributing said finding of deviation or no deviation to a particular diagnosis, prediction and/or prognosis of impaired bone fracture healing in the subject.

6. The method for monitoring impaired bone fracture healing or for monitoring the risk of developing impaired bone fracture healing in a subject according to claim 4 comprising steps: (i) measuring the quantity of IL-8 in a sample from the subject at two or more successive time points; (ii) comparing the quantity as measured in (i) between said two or more successive time points; (iii) finding a deviation or no deviation of the quantity as measured in (i) between said two or more successive time points; (iv) attributing said finding of deviation or no deviation to a change in impaired bone fracture healing or to a change in the risk of developing impaired bone fracture healing in the subject between the two or more successive time points.

7. A method to determine whether a subject is or is not in need of a therapeutic or prophylactic treatment of impaired bone fracture healing comprising measuring the level of IL-8 in a sample from said subject, preferably comprising steps: (i) measuring the quantity of IL-8 in the sample from the subject; (ii) comparing the quantity as measured in (i) with a reference value representing a known diagnosis, prediction and/or prognosis of impaired bone fracture healing; (iii) finding a deviation or no deviation of the quantity as measured in (i) from the reference value; (iv) inferring from said finding the presence or absence of a need for a therapeutic or prophylactic treatment of impaired bone fracture healing.

8. The method according to any one of claims 4 to 7, wherein the impaired bone fracture healing is selected from the group consisting of mal-union fracture, delayed union fracture, and non-union fracture.

9. The use according to any one of claims 1 to 3 or the method according to any one of claims 4 to 8, further comprising measuring the level of any one or more of SDF-1, PDGF-BB, IL-6, TGF-β, PDGF, FGF and BMP in a sample from the subject.

10. A method for establishing a reference value for IL-8, said reference value representing:
(a) a prediction or diagnosis of the absence of impaired bone fracture healing or a good prognosis for impaired bone fracture healing, or
(b) a prediction or diagnosis of impaired bone fracture healing or a poor prognosis for impaired bone fracture healing,
comprising:
(i) measuring the quantity of IL-8 in a sample from:
(i a) one or more subjects not having impaired bone fracture healing or not being at risk of having impaired bone fracture healing or having a good prognosis for impaired bone fracture healing, or
(i b) one or more subjects having impaired bone fracture healing or being at risk of having impaired bone fracture healing or having a poor prognosis for impaired bone fracture healing, and
(ii a) establishing from the quantity of IL-8 as measured in (i a) the reference value representing the prediction or diagnosis of the absence of impaired bone fracture healing or representing the good prognosis for impaired bone fracture healing, or
(ii b) establishing from the quantity of IL-8 as measured in (i b) the reference value representing the prediction or diagnosis of impaired bone fracture healing or representing the poor prognosis for impaired bone fracture healing.

11. The use according to any one of claims 1 to 3 or 9 or the method according to any one of claims 4 to 10, comprising measuring the systemic quantity of IL-8, preferably wherein the sample comprises, consists essentially of or consists of whole blood or a fractional component thereof, more preferably plasma or serum.

12. The use according to any one of claims 1 to 3 or 9 or the method according to any one of claims 4 to 10, comprising measuring the quantity of IL-8 in cells or in the supernatant of cells obtained from the subject and subsequently cultured *in vitro.*

13. The use or method according to claim 12, wherein the cells are osteoblasts (OB) or mesenchymal stem cells (MSC).

14. Use of a kit for the diagnosis, prediction, prognosis and/or monitoring of impaired bone fracture healing in a subject, the kit comprising (i) one or more binding agents capable of specifically binding to IL-8, particularly in a sample from the subject, and (ii) optionally and preferably one or more reference values or means for establishing said one or more reference values, wherein said one or more reference values represent a known diagnosis, prediction and/or prognosis of impaired bone fracture healing.

15. The use according to claim 14, wherein means for collecting a sample from a subject is provided separately from or is comprised in the kit.

16. Use of a nucleic acid array or microarray or a protein, polypeptide or peptide array or microarray for the diagnosis, prediction, prognosis and/or monitoring of impaired bone fracture healing, said array or microarray comprising a nucleic acid encoding IL-8 or comprising IL-8.

17. Use of a binding agent array or microarray for the diagnosis, prediction, prognosis and/or monitoring of impaired bone fracture healing, said array or microarray comprising one or more binding agents capable of specifically binding to IL-8, preferably comprising a known quantity or concentration of said one or more binding agents.

## Patentansprüche

1. Verwendung von Interleukin-8 (IL-8) als Biomarker für beeinträchtigte Knochenbruchheilung.

2. Verwendung von IL-8 zur Diagnose, Vorhersage, Prognose und/oder Überwachung von beeinträchtigter Knochenbruchheilung.

3. Verwendung nach einem der Ansprüche 1 oder 2, wobei die beeinträchtigte Knochenbruchheilung ausgewählt ist aus der Gruppe, bestehend aus Bruchfehlstellungsheilung, verzögerter Bruchheilung und Nichtvereinigung der Bruchenden.

4. Verfahren zur Diagnose, Vorhersage, Prognose und/oder Überwachung von beeinträchtigter Knochenbruchheilung in einem Individuum, umfassend das Messen des IL-8-Spiegels in einer Probe aus dem Individuum.

5. Verfahren zur Diagnose, Vorhersage und/oder Prognose von beeinträchtigter Knochenbruchheilung in einem Individuum nach Anspruch 4, umfassend die Schritte: (i) Messen der Menge IL-8 in einer Probe aus dem Individuum; (ii) Vergleichen der wie in (i) gemessenen Menge mit einem Referenzwert, der eine bekannte Diagnose, Vorhersage und/oder Prognose von beeinträchtigter Knochenbruchheilung darstellt; (iii) Ermitteln einer Abweichung oder keiner Abweichung der wie in (i) gemessenen Menge von dem Referenzwert; und (iv) Zuordnen des Befundes von Abweichung oder keiner Abweichung zu einer bestimmten Diagnose, Vorhersage und/oder Prognose von beeinträchtigter Knochenbruchheilung in dem Individuum.

6. Verfahren zur Überwachung von beeinträchtigter Knochenbruchheilung bzw. zur Überwachung der Gefahr der Entwicklung von beeinträchtigter Knochenbruchheilung in einem Individuum nach Anspruch 4, umfassend die Schritte: (i) Messen der Menge IL-8 in einer Probe aus dem Individuum an zwei oder mehr aufeinander folgenden Zeitpunkten; (ii) Vergleichen der wie in (i) gemessenen Menge zwischen den zwei oder mehr aufeinanderfolgenden Zeitpunkten; (iii) Ermitteln einer Abweichung oder keiner Abweichung der wie in (i) gemessenen Menge, zwischen den zwei oder mehreren aufeinanderfolgenden Zeitpunkten; (iv) Zuordnen des Befundes von Abweichung oder keiner Abweichung zu einer Änderung bei der beeinträchtigten Knochenbruchheilung oder einer Änderung bei der Gefahr zur Entwicklung von beeinträchtigter Knochenbruchheilung in dem Individuum zwischen den zwei oder mehr aufeinanderfolgenden Zeitpunkten.

7. Verfahren zur Bestimmung, ob ein Patient eine therapeutische oder prophylaktische Behandlung von beeinträchtigter Knochenbruchheilung benötigt oder nicht, umfassend das Messen des IL-8-Spiegels in einer Probe aus dem Individuum, umfassend vorzugsweise die folgenden Schritte: (i) Messen der Menge IL-8 in der Probe aus dem Individuum; (ii) Vergleichen der wie in (i) gemessenen Menge mit einem Referenzwert, der eine bekannte Diagnose, Vorhersage und/oder Prognose von beeinträchtigter Knochenbruchheilung darstellt; (iii) Ermitteln einer Abweichung oder keiner Abweichung der wie in (i) gemessenen Menge von dem Referenzwert; (iv) Ableiten des Vorhandenseins oder Fehlens einer Notwendigkeit für eine therapeutische oder prophylaktische Behandlung von beeinträchtigter Knochenbruchheilung aus dem Befund.

8. Verfahren nach einem der Ansprüche 4 bis 7, wobei die beeinträchtigte Knochenbruchheilung ausgewählt ist aus der Gruppe, bestehend aus Bruchfehlstellungsheilung, verzögerter Bruchheilung und Nichtvereinigung der Bruchenden.

9. Verwendung nach einem der Ansprüche 1 bis 3 oder das Verfahren nach einem der Ansprüche 4 bis 8, zudem umfassend das Messen der Menge von einem oder mehreren von SDF-1, PDGF-BB, IL-6, TGF-β, PDGF, FGF und BMP in einer Probe aus dem Individuum.

10. Verfahren zur Festlegung eines Referenzwerts für IL-8, wobei der Referenzwert darstellt:
(a) eine Vorhersage oder Diagnose der Abwesenheit von beeinträchtigter Knochenbruchheilung oder eine gute Prognose für beeinträchtigte Knochenbruchheilung oder
(b) eine Vorhersage oder Diagnose von beeinträchtigter Knochenbruchheilung oder eine schlechte Prognose für beeinträchtigte Knochenbruchheilung, umfassend:
(i) Messen der Menge IL-8 in einer Probe aus:
(i a) einem oder mehreren Individuen, die keine beeinträchtigte Knochenbruchheilung aufweisen oder nicht der Gefahr unterliegen, eine beeinträchtigte Knochenbruchheilung zu haben oder eine gute Prognose für beeinträchtigte Knochenbruchheilung haben oder
(i b) einem oder mehreren Individuen, die eine eingeschränkte Knochenbruchheilung aufweisen oder der Gefahr unterliegen, eine beeinträchtigte Knochenbruchheilung zu haben oder eine schlechte Prognose für beeinträchtigte Knochenbruchheilung haben und
(ii a) Festlegen des Referenzwerts, der die Vorhersage oder Diagnose der Abwesenheit von beeinträchtigter Knochenbruchheilung darstellt oder die gute Prognose für beeinträchtigte Knochenbruchheilung darstellt, aus der wie in (i a) gemessenen Menge IL-8, oder
(ii b) Festlegen des Referenzwerts, der die Vorhersage oder Diagnose von beeinträchtigter Knochenbruchheilung darstellt oder die schlechte Prognose für beeinträchtigte Knochenbruchheilung darstellt, aus der wie in (i b) gemessenen Menge IL-8.

11. Verwendung nach einem der Ansprüche 1 bis 3 oder 9 oder Verfahren nach einem der Ansprüche 4 bis 10, umfassend das Messen der systemischen Menge IL-8, wobei die Probe vorzugsweise Vollblut oder eine Teilkomponente davon, stärker bevorzugt Plasma oder Serum umfasst, im Wesentlichen daraus besteht oder daraus besteht.

12. Verwendung nach einem der Ansprüche 1 bis 3 oder 9 oder Verfahren nach einem der Ansprüche 4 bis 10, umfassend das Messen der Menge IL-8 in Zellen oder im Überstand von Zellen, die aus dem Individuum erhalten wurden und anschließend *in vitro* gezüchtet wurden.

13. Verwendung oder Verfahren nach Anspruch 12, wobei die Zellen Osteoblasten (OB) oder mesenchymale Stammzellen (MSC) sind.

14. Verwendung eines Kits zur Diagnose, Vorhersage, Prognose und/oder Überwachung von beeinträchtigter Knochenbruchheilung in einem Individuum, wobei das Kit umfasst: (i) ein oder mehrere Bindemittel, die spezifisch an IL-8 binden können, insbesondere in einer Probe aus dem Individuum, und (ii) gegebenenfalls und vorzugsweise einen oder mehrere Referenzwerte oder Mittel zum Bestimmen der ein oder mehreren Referenzwerte, wobei die ein oder mehreren Referenzwerte eine bekannte Diagnose, Vorhersage und/oder Prognose von beeinträchtigter Knochenbruchheilung darstellen.

15. Verwendung nach Anspruch 14, wobei Mittel zum Sammeln einer Probe aus einem Individuum getrennt von dem Kit bereitgestellt werden oder in diesem enthalten sind.

16. Verwendung eines Nukleinsäure-Arrays oder Mikroarrays oder eines Protein-, Polypeptid- oder Peptid-Arrays oder Mikroarrays zur Diagnose, Vorhersage, Prognose und/oder Überwachung von beeinträchtigter Knochenbruchheilung, wobei das Array oder Mikroarray eine Nukleinsäure umfasst, die IL-8 codiert, oder IL-8 umfasst.

17. Verwendung eines Bindemittel-Arrays oder Mikroarrays zur Diagnose, Vorhersage, Prognose und/oder Überwachung von beeinträchtigter Knochenbruchheilung, wobei das Array oder Mikroarray ein oder mehrere Bindemittel umfasst, die spezifisch an IL-8 binden können, vorzugsweise eine bekannte Menge oder Konzentration der ein oder mehreren Bindemittel umfasst.

## Revendications

1. Utilisation de l'interleukine-8 (IL-8) en tant que biomarqueur pour la cicatrisation de fracture osseuse altérée.

2. Utilisation d'IL-8 pour le diagnostic, la prédiction, le pronostic et/ou la surveillance d'une cicatrisation de fracture osseuse altérée.

3. Utilisation selon l'une quelconque des revendications 1 ou 2, dans laquelle la cicatrisation de fracture osseuse altérée est choisie dans le groupe constitué d'un défaut de fusion de fracture, une fracture à fusion retardée, et une fracture sans fusion.

4. Procédé pour le diagnostic, la prédiction, le pronostic et/ou la surveillance de cicatrisation de fracture osseuse altérée chez un sujet comprenant la mesure du taux d'IL-8 dans un échantillon dudit sujet.

5. Procédé de diagnostic, prédiction et/ou pronostic de cicatrisation de fracture osseuse altérée chez un sujet selon la revendication 4 comprenant les étapes de : (i) mesure de la quantité d'IL-8 chez un échantillon du sujet ; (ii) comparaison de la quantité telle que mesurée dans (i) à une valeur de référence représentant un diagnostic, une prédiction et/ou un pronostic connus de cicatrisation de fracture osseuse altérée ; (iii) observation d'un écart ou d'une absence d'écart de la quantité telle que mesurée dans (i) par rapport à la valeur de référence ; et (iv) attribution de ladite observation d'écart ou d'absence d'écart par rapport à un diagnostic, une prédiction et/ou un pronostic particuliers de cicatrisation de fracture osseuse altérée chez le sujet.

6. Procédé de surveillance de cicatrisation de fracture osseuse altérée ou de surveillance du risque de développement de cicatrisation de fracture osseuse altérée chez un sujet selon la revendication 4 comprenant les étapes de : (i) mesure de la quantité d'IL-8 dans un échantillon du sujet à deux temps successifs ou plus ; (ii) comparaison de la quantité telle que mesurée dans (i) entre lesdits deux temps successifs ou plus ; (iii) observation d'un écart ou d'une absence d'écart de la quantité telle que mesurée dans (i) entre lesdits deux temps successifs ou plus ; (iv) attribution de ladite observation d'écart ou d'absence d'écart par rapport à un changement de cicatrisation de fracture osseuse altérée ou un changement du risque de développement de cicatrisation de fracture osseuse altérée chez le sujet entre les deux temps successifs ou plus.

7. Procédé de détermination du fait qu'un sujet nécessite ou non un traitement thérapeutique ou prophylactique de cicatrisation de fracture osseuse altérée comprenant la mesure du taux d'IL-8 dans un échantillon dudit sujet, comprenant de préférence les étapes de : (i) mesure de la quantité d'IL-8 dans l'échantillon du sujet ; (ii) comparaison de la quantité telle que mesurée dans (i) à une valeur de référence représentant des diagnostic, prédiction et/ou pronostic connus de cicatrisation de fracture osseuse altérée ; (iii) observation d'un écart ou de l'absence d'écart de la quantité telle que mesurée dans (i) par rapport à la valeur de référence ; (iv) déduction de ladite observation de la présence ou l'absence d'une nécessité d'un traitement thérapeutique ou prophylactique de cicatrisation de fracture osseuse altérée.

8. Procédé selon l'une quelconque des revendications 4 à 7, dans lequel la cicatrisation de fracture osseuse altérée est choisie dans le groupe constitué d'un défaut de fusion de fracture, une fusion de fracture retardée, et une fracture sans fusion.

9. Utilisation selon l'une quelconque des revendications 1 à 3 ou procédé selon l'une quelconque des revendications 4 à 8, comprenant en outre la mesure d'un ou plusieurs quelconques de SDF-1, PDGF-BB, IL-6, TGF-β, PDGF, FGF et BMP dans un échantillon du sujet.

10. Procédé d'établissement d'une valeur de référence pour IL-8, ladite valeur de référence représentant :
(a) une prédiction ou un diagnostic de l'absence de cicatrisation de fracture osseuse altérée ou un pronostic positif de cicatrisation de fracture osseuse altérée, ou
(b) une prédiction ou un diagnostic de cicatrisation de fracture osseuse altérée ou un pronostic médiocre de cicatrisation de fracture osseuse altérée,
comprenant :
(i) la mesure de la quantité d'IL-8 dans un échantillon de :
(ia) un ou plusieurs sujets n'ayant pas de cicatrisation de fracture osseuse altérée ou n'étant pas à risque de présenter une cicatrisation de fracture osseuse altérée ou ayant un pronostic positif de cicatrisation de fracture osseuse altérée, ou
(ib) un ou plusieurs sujets ayant une cicatrisation de fracture osseuse altérée ou étant à risque de présenter une cicatrisation de fracture osseuse altérée ou ayant un pronostic médiocre de cicatrisation de fracture osseuse altérée, et
(iia) établissement à partir de la quantité d'IL-8 telle que mesurée dans (ia) de la valeur de référence représentant la prédiction ou le diagnostic de l'absence de cicatrisation de fracture osseuse altérée ou représentant le pronostic médiocre de cicatrisation de fracture osseuse altérée, ou
(iib) établissement à partir de la quantité d'IL-8 telle que mesurée dans (ib) de la valeur de référence représentant la prédiction ou le diagnostic de cicatrisation de fracture osseuse altérée ou représentant le pronostic médiocre de cicatrisation de fracture osseuse altérée.

11. Utilisation selon l'une quelconque des revendications 1 à 3 ou 9 ou procédé selon l'une quelconque des revendications 4 à 10, comprenant la mesure de la quantité systémique d'IL-8, de préférence où l'échantillon comprend, est essentiellement constitué de ou est constitué de sang total ou d'un composant fractionnaire de celui-ci, plus préférablement du plasma ou du sérum.

12. Utilisation selon l'une quelconque des revendications 1 à 3 ou 9 ou procédé selon l'une quelconque des revendications 4 à 10, comprenant la mesure de la quantité d'IL-8 dans des cellules ou dans le surnageant de cellules obtenues à partir du sujet et ensuite cultivées *in vitro.*

13. Utilisation ou procédé selon la revendication 12, dans lesquels les cellules sont des ostéoblastes (OB) ou des cellules souches mésenchymateuses (MSC).

14. Utilisation d'un kit pour le diagnostic, la prédiction, le pronostic et/ou la surveillance de la cicatrisation de fracture osseuse altérée chez un sujet, le kit comprenant (i) un ou plusieurs agents de liaison capables de se lier spécifiquement à IL-8, en particulier dans un échantillon du sujet, et (ii) facultativement et de préférence une ou plusieurs valeurs de référence ou des moyens pour établir lesdites une ou plusieurs valeurs de référence, où lesdites une ou plusieurs valeurs de référence représentent un diagnostic, une prédiction et/ou pronostic de cicatrisation de fracture osseuse altérée.

15. Utilisation selon la revendication 14, dans laquelle des moyens pour collecter un échantillon d'un sujet sont disposés séparément de ou sont compris dans le kit.

16. Utilisation d'une puce ou micropuce d'acide nucléique ou puce ou micropuce de protéine, polypeptide ou peptide pour le diagnostic, la prédiction, le pronostic et/ou la surveillance de cicatrisation de fracture osseuse altérée, ladite puce ou micropuce comprenant un acide nucléique codant pour IL-8 ou comprenant IL-8.

17. Utilisation d'une puce ou micropuce d'agent de liaison pour le diagnostic, la prédiction, le pronostic et/ou la surveillance de la cicatrisation de fracture osseuse altérée, ladite puce ou micropuce comprenant un ou plusieurs agents de liaison capables de se lier spécifiquement à IL-8, comprenant de préférence une quantité ou concentration connue desdits un ou plusieurs agents de liaison.
